# EUROPEAN PATENT APPLICATION

(11) **EP 4 349 391 A1**
(43) Date of publication of application: **10.04.2024**
(21) Application number: 22811563.0
(22) Date of filing: 18.05.2022
(51) Int. Cl.: A61M 25/01, A61M 25/09, A61B 34/30, A61B 34/00, A61M 25/00

(54) **DEVICE FOR VASCULAR INTERVENTIONAL PROCEDURE**

(30) Priority: 26.05.2021 KR 20210067774; 21.02.2022 KR 20220022298
(71) Applicant: Perazah Inc., Ansan-si, Gyeonggi-do 15588 (KR)
(72) Inventor: SEO, Jong Tae, Ansan-si Gyeonggi-do 15588 (KR)
(74) Representative: Cabinet Nony
(86) International application number: PCT/KR2022/007130
(87) International publication number: WO 2022/250370

(57) **Abstract**

A vascular intervention procedure device is provided. The vascular intervention procedure device includes a base frame, a transfer module, and first and second procedure tool modules. The transfer module is transferred in a front and rear direction along the base frame. The first procedure tool module is coupled to the transfer module so as to be transferred with respect to the base frame in the front and rear direction, and rotates a first procedure tool, which is a catheter, about a rotation axis of the front and rear direction. The second procedure tool module is coupled to the transfer module so as to be transferred with respect to the base frame in the front and rear direction independently of the first procedure tool module, and supports a second procedure tool which is a guide wire or a micro catheter inserted into the catheter.

## Description

### TECHNICAL FIELD

The present disclosure relates to a vascular intervention procedure device that transfers and/or rotates a procedure tool for a vascular intervention procedure.

### BACKGROUND ART

An intervention procedure is a treatment method that treats specific diseases through percutaneous, minimally invasive manipulations using various tools such as a guide wire, a catheter, a balloon, and a stent under various imaging apparatuses. The intervention procedure does not require a large surgical incision. The intervention procedure has advantages such as reduced bleeding due to a minimal skin incision, rapid patient recovery, and nonoccurrence of complications due to local anesthesia. The intervention procedure may be classified into a nonvascular procedure and a vascular procedure.

A vascular intervention procedure may be used for treatment of blood vessel diseases and cancers. The vascular intervention procedure involves access to a target organ by percutaneous insertion of a catheter having a diameter of 1 mm to 7 mm through a blood vessel under ultrasound or fluoroscopic guidance. The vascular intervention procedure may be applied to liver tumors, arterial bleeding, vascular occlusion or stenosis, and uterine fibroid embolization, and its scope of application is currently expanding.

By way of example, in the vascular intervention procedure, a blood vessel is punctured under ultrasound guidance or by artery palpation, and an introducer sheath is inserted and fixed to protect the blood vessel from a trauma. Thereafter, procedure tools such as the guide wire and the catheter are inserted into the blood vessel through the introducer sheath, and the guide wire and the catheter are manipulated to select the target blood vessel. The guide wire reaches the target blood vessel by the manipulation of insertion and rotation. Subsequently, the catheter is inserted into the target blood vessel along the guide wire. To make the catheter reach the target blood vessel, the insertion and rotation of the guide wire and the insertion and rotation of the catheter may be repeated. In order to access a smaller blood vessel, a micro catheter having a diameter smaller than the diameter of the catheter, and a micro guide wire are used, and the micro catheter and the micro guide wire are inserted into the catheter. The direction to the target blood vessel is determined by the insertion and rotation of the micro guide wire, and then the micro catheter is inserted along the micro guide wire. After the guide wire or the micro guide wire is removed, a medicament or a therapeutic medicine is injected into the target blood vessel through the catheter or the micro catheter.

Since the vascular intervention procedure is generally performed under fluoroscopy (X-ray radiography) guidance, an operator is exposed to X-ray radiation. Since the operator in a vascular intervention procedure room wears a heavy clothing and device for shielding radiation, the operator suffers a lot of physical stresses. Since the operator frequently enters and exits the procedure room due to the radiation exposure as compared to a regular operating room, the vascular intervention procedure room may show a poor degree of disinfection state and cleanliness state. Since the manipulation of the catheter and the guide wire in the vascular intervention procedure relies on the experience and skill of the operator, it may not be possible to perform accurate and precise manipulation.

### DETAILED DESCRIPTION OF THE INVENTION

### Technical Problem

The embodiments of the present disclosure solve the problems of the vascular intervention procedure due to the aforementioned operator's manipulation. The embodiments of the present disclosure provide a vascular intervention procedure device which inserts and/or rotates procedure tools used for a vascular intervention procedure. The embodiments of the present disclosure provide a vascular intervention procedure device having enhanced operability and usability.

### Means for Solving the Problem

A vascular intervention procedure device of one embodiment includes a base frame, a transfer module, a first procedure tool module, and a second procedure tool module. The base frame extends in a front and rear direction. The transfer module includes a transfer frame coupled to the base frame so as to be transferred in the front and rear direction along the base frame. The first procedure tool module is coupled to the transfer module so as to be transferred with respect to the base frame in the front and rear direction, and is configured to rotate a first procedure tool, which is a catheter, about a rotation axis of the front and rear direction. The second procedure tool module is coupled to the transfer module so as to be transferred with respect to the base frame in the front and rear direction independently of the first procedure tool module, and is configured to support a second procedure tool which is a guide wire or a micro catheter inserted into the catheter.

In one embodiment, the transfer module includes a first transferring portion and a second transferring portion. The first transferring portion is disposed in the transfer frame. The first transferring portion is configured to be coupled to the first procedure tool module and to transmit rotation power for rotating the first procedure tool to the first procedure tool module. The second transferring portion is disposed in the transfer frame so as to be transferred independently of the first transferring portion in rear of the first transferring portion, and is coupled to the second procedure tool module.

In one embodiment, the transfer module includes a module transferring lead screw, which is disposed along the front and rear direction and is coupled to the second transferring portion so as to transfer the second transferring portion through screw motion.

In one embodiment, the second procedure tool module is a micro catheter module configured to support the micro catheter. The vascular intervention procedure device may further include a third procedure tool module. The third procedure tool module is coupled to the transfer module so as to be transferred with respect to the base fame in the front and rear direction, and is configured to rotate a third procedure tool, which is a micro guide wire inserted into the micro catheter, about the rotation axis.

In one embodiment, the transfer module includes the first transferring portion, the second transferring portion, and the third transferring portion. The first transferring portion is disposed in the transfer frame. The first transferring portion is configured to be coupled to the first procedure tool module and to transmit the rotation power for rotating the first procedure tool to the first procedure tool module. The second transferring portion is disposed in the transfer frame so as to be transferred independently of the first transferring portion in rear of the first transferring portion, and is coupled to the second procedure tool module. The third transferring portion is disposed in the transfer frame so as to be transferred in the front and rear direction independently of the first transferring portion and the second transferring portion in rear of the second transferring portion. The third transferring portion is configured to be coupled to the third procedure tool module and to transmit rotation power for rotating the third procedure tool to the third procedure tool module.

In one embodiment, the transfer module includes the module transferring lead screw, which is disposed along the front and rear direction and is coupled to the second transferring portion and the third transferring portion so as to transfer the second transferring portion and the third transferring portion through screw motion. The second transferring portion includes a second transferring nut coupled to the module transferring lead screw so as to be transferred through screw motion, and a second transferring motor configured to rotate the second transferring nut. The third transferring portion includes a third transferring nut coupled to the module transferring lead screw so as to be transferred through screw motion, and a third transferring motor configured to rotate the third transferring nut.

In one embodiment, the first transferring portion may include a transferring nut coupled to the module transferring lead screw so as to be transferred through screw motion.

In one embodiment, the first transferring portion includes a rotation power generating portion generating rotation power for rotating the first procedure tool, and a power transmitting portion configured to be connected to the rotation power generating portion, to be coupled to the first procedure tool module, and to transmit the rotation power for rotating the first procedure tool.

In one embodiment, the first transferring portion is disposed in the transfer frame so as to be transferable in the front and rear direction, and the transfer module may be configured to transfer the first transferring portion and the second transferring portion simultaneously or independently.

In one embodiment, the second procedure tool module may be configured to rotate the second procedure tool about the rotation axis, and the second transferring portion may be configured to transmit rotation power for rotating the second procedure tool to the second procedure tool module.

In one embodiment, the vascular intervention procedure device may include a guide module disposed in a guide section, which is a section between a front end of the base frame and the first procedure tool module or a section between the first procedure tool module and the second procedure tool module, and configured to guide and support transfer of the first procedure tool or the second procedure tool in the front and rear direction. The guide module includes a guide housing and a pair of supporting members. The guide housing is disposed at a front end or a rear end of the guide section. The pair of supporting members are configured to be drawn into or drawn out from the guide housing so as to have a variable length in the guide section according to a length change of the guide section in the front and rear direction and are configured to sandwich the first procedure tool or the second procedure tool by being engaged with each other.

In one embodiment, the guide module includes an engaging portion disposed in the guide housing and configured to make contact with each of the pair of supporting members and to cause the pair of supporting members to be engaged with each other when the pair of supporting members are drawn out from the guide housing.

In one embodiment, the engaging portion includes: a pair of engaging wheels rotated by making contact with the pair of supporting members, respectively, and causing the pair of supporting members to be engaged with each other; and a pair of springs pressing the pair of engaging wheels, respectively, in a direction where the pair of supporting members are drawn into the guide housing.

In one embodiment, the guide housing includes a pair of spring shafts coupled to the pair of springs, respectively, and configured to adjust restoring forces of the pair of springs, respectively.

In one embodiment, the guide module includes a splitting portion disposed between the pair of supporting members in the guide housing and configured to split the pair of supporting members when the pair of supporting members are drawn into the guide housing.

In one embodiment, the pair of supporting members may include a first chain assembly having engagement teeth and engagement grooves which are disposed alternately, and a second chain assembly having engagement grooves and engagement teeth which correspond to the engagement teeth and the engagement grooves of the first chain assembly, respectively.

In one embodiment, the pair of supporting members may include a first band having an engagement protrusion formed in the front and rear direction, and a second band having an engagement groove into which the engagement protrusion is fitted.

In one embodiment, the guide module may include a first guide module and a second guide module. The first guide module is disposed in a first guide section between the front end of the base frame and the first procedure tool module and is configured to guide and support transfer of the first procedure tool in the front and rear direction. The second guide module is disposed in a second guide section between the first procedure tool module and the second procedure tool module and is configured to guide and support transfer of the second procedure tool in the front and rear direction.

In one embodiment, the vascular intervention procedure device includes the guide module disposed in the guide section, which is a section between a front end of the base frame and the first procedure tool module, a section between the first procedure tool module and the second procedure tool module, or a section between the second procedure tool module and the third procedure tool module, and configured to guide and support transfer of the first procedure tool, the second procedure tool, or the third procedure tool in the front and rear direction. The guide module includes the guide housing and a pair of supporting members. The guide housing is disposed at a front end or a rear end of the guide section. The pair of supporting members are configured to be drawn into or drawn out from the guide housing so as to have a variable length in the guide section according to a length change of the guide section in the front and rear direction and to sandwich the first procedure tool, the second procedure tool, or the third procedure tool by being engaged with each other.

In one embodiment, the vascular intervention procedure device includes a transfer module driving portion transferring the transfer module with respect to the base frame in the front and rear direction. The transfer module may be slidably coupled to the base frame by the transfer module driving portion.

### Effect of the Invention

According to one embodiment of the present disclosure, multi degree-of-freedom operations of a plurality of procedure tools are realized, and the operability and usability of the vascular intervention procedure device can be enhanced thereby.

According to one embodiment of the present disclosure, due to the base platform and a plurality of procedure tool modules, a complicated vascular intervention procedure can be performed by means of a single vascular intervention procedure device.

### BRIEF DESCRIPTION OF DRAWINGS

FIG. 1 schematically shows an example of a vascular intervention procedure that is performed using a catheter and a guide wire by a vascular intervention procedure device according to one embodiment.
FIG. 2 schematically shows another example of the vascular intervention procedure that is performed using a catheter, a micro catheter, and a micro guide wire by a vascular intervention procedure device according to one embodiment.
FIG. 3 schematically shows an example of the state where a vascular intervention procedure device according to one embodiment is used for the vascular intervention procedure.
FIG. 4 is a perspective view showing a vascular intervention procedure device according to one embodiment in one operation mode.
FIG. 5 is a perspective view showing a vascular intervention procedure device according to one embodiment in another operation mode.
FIG. 6 is a perspective view showing a platform of a vascular intervention procedure device according to one embodiment.
FIG. 7 is an exploded perspective view showing a base frame of the platform shown in FIG. 6.
FIG. 8 is a plan view showing the base frame of the platform shown in FIG. 6.
FIG. 9 is a cross-sectional view taken along line IX-IX of FIG. 8.
FIG. 10 is an exploded perspective view showing a transfer frame of the platform shown in FIG. 6.
FIG. 11 is a cross-sectional view taken along line XI-XI of FIG. 10.
FIG. 12 is a sectional perspective view taken along line XII-XII of FIG. 10.
FIG. 13 is a perspective view showing an example of a transferring portion shown in FIG. 10.
FIG. 14 is a lower perspective view showing a power transmitting portion of the transferring portion shown in FIG. 13.
FIG. 15 is a cross-sectional view taken along line XV-XV of FIG. 14.
FIG. 16 is a cross-sectional view showing a state before a rotation power generating portion and a power transferring portion of the transferring portion shown in FIG. 13 are coupled to each other.
FIG. 17 is a cross-sectional view showing a state where the rotation power generating portion and the power transferring portion of the transferring portion shown in FIG. 13 are coupled to each other.
FIG. 18 is a perspective view showing a state where the power transmitting portion of the transferring portion and a procedure tool module are coupled to each other.
FIG. 19 is a perspective view showing a catheter module of the vascular intervention procedure device according to one embodiment.
FIG. 20 is a cross-sectional view taken along line XX-XX of FIG. 19.
FIG. 21 is a lower exploded perspective view of the catheter module shown in FIG. 19.
FIG. 22 is a perspective view showing a guide wire module of the vascular intervention procedure device according to one embodiment.
FIG. 23 is a lower exploded perspective view of the guide wire module shown in FIG. 22.
FIG. 24 is a cross-sectional view taken along line XXIV-XXIV of FIG. 22.
FIG. 25 is an exploded perspective view showing a guide wire clamp shown in FIG. 24.
FIG. 26 is a perspective view showing a micro catheter module of the vascular intervention procedure device according to one embodiment.
FIG. 27 is a cross-sectional view taken along line XXVII-XXVII of FIG. 26.
FIG. 28 is a perspective view showing a micro guide wire module of the vascular intervention procedure device according to one embodiment.
FIG. 29 is a cross-sectional view taken along line XXIX-XXIX of FIG. 28.
FIG. 30 is a perspective view showing a first guide module of the vascular intervention procedure device according to one embodiment.
FIG. 31 is an exploded perspective view of the first guide module shown in FIG. 30.
FIG. 32 is a lower exploded perspective view showing a first guide housing of the first guide module shown in FIG. 30.
FIG. 33 is a perspective view showing an example where the first guide module shown in FIG. 30 is coupled to a front end of a base frame.
FIG. 34 is a perspective view showing a second guide module of the vascular intervention procedure device according to one embodiment.
FIG. 35 is an exploded perspective view of the second guide module shown in FIG. 34.
FIG. 36 is a lower exploded perspective view of the second guide module shown in FIG. 34.
FIG. 37 is a cross-sectional view taken along line XXXVII-XXXVII of FIG. 34.
FIG. 38 is a perspective view showing the catheter module and the second guide module of the vascular intervention procedure device according to one embodiment.
FIG. 39 is a perspective view showing a ready state before operation in a first operation mode of the vascular intervention procedure device according to one embodiment.
FIG. 40 is a perspective view showing a state where procedure tool modules are fitted to the transferring portions for the first operation mode of the vascular intervention procedure device according to one embodiment.
FIG. 41 is a perspective view showing a state where the first guide module is coupled to a front end of the platform for the first operation mode of the vascular intervention procedure device according to one embodiment.
FIG. 42 is a perspective view showing an operation subsequent to the state shown in FIG. 41.
FIG. 43 is a perspective view showing an operation subsequent to the state shown in FIG. 42.
FIG. 44 is a perspective view showing an operation subsequent to the state shown in FIG. 43.
FIG. 45 is a perspective view showing an operation subsequent to the state shown in FIG. 44.
FIG. 46 is a perspective view showing an example where the guide wire module is removed in the first operation mode of the vascular intervention procedure device according to one embodiment.
FIG. 47 is a perspective view showing an initial state in a second operation mode of the vascular intervention procedure device according to one embodiment.
FIG. 48 is a perspective view showing a state where the micro catheter module and the micro guide wire module are coupled to the transfer frame of the platform for the second operation mode of the vascular intervention procedure device according to one embodiment.
FIG. 49 is a perspective view showing a state where the second guide module is coupled to the micro catheter module for the second operation mode of the vascular intervention procedure device according to one embodiment.
FIG. 50 is a perspective view showing an operation subsequent to the state shown in FIG. 49.
FIG. 51 is a perspective view showing an operation subsequent to the state shown in FIG. 50.
FIG. 52 is a perspective view showing an operation subsequent to the state shown in FIG. 51.
FIG. 53 schematically shows a configuration of a vascular intervention procedure device according another embodiment.
FIG. 54 is a cross-sectional view showing a micro catheter module of the vascular intervention procedure device shown in FIG. 53.
FIG. 55 is a cross-sectional view showing a guide wire module of the vascular intervention procedure device shown in FIG. 53.
FIG. 56 is a plan view showing a first guide module of the vascular intervention procedure device shown in FIG. 53.
FIG. 57 is a perspective showing a first guide module and a second guide module of the vascular intervention procedure device according another embodiment.
FIG. 58 shows a cross-sectional shape of the second guide module shown in FIG. 57.

### MODES FOR CARRYING OUT THE INVENTION

Embodiments of the present disclosure are illustrated for the purpose of explaining the technical idea of the present disclosure. The scope of the rights according to the present disclosure is not limited to the embodiments presented below or the detailed descriptions of such embodiments.

All the technical terms and scientific terms used in the present disclosure include meanings that are commonly understood by those of ordinary skill in the technical field to which the present disclosure pertains unless otherwise defined. All terms used in the present disclosure are selected for the purpose of describing the present disclosure more clearly, and are not selected to limit the scope of the rights according to the present disclosure.

Expressions such as "comprising," "including," "having," and the like used in the present disclosure are to be understood as open-ended terms having the possibility of encompassing other embodiments, unless otherwise mentioned in the phrase or sentence containing such expressions.

Singular expressions that are described in the present disclosure may encompass plural expressions unless otherwise stated, which will also be applied to the singular expressions recited in the claims.

Expressions such as "first," "second," etc. used in the present disclosure are used to distinguish a plurality of elements from each other, and are not intended to limit an order or importance of the elements.

In the present disclosure, the description that one element is "connected" or "coupled" to another element should be understood to indicate that one element may be directly connected, or coupled, to another element, or that a new different element may be interposed between the one element and the another element to be connected or coupled thereby.

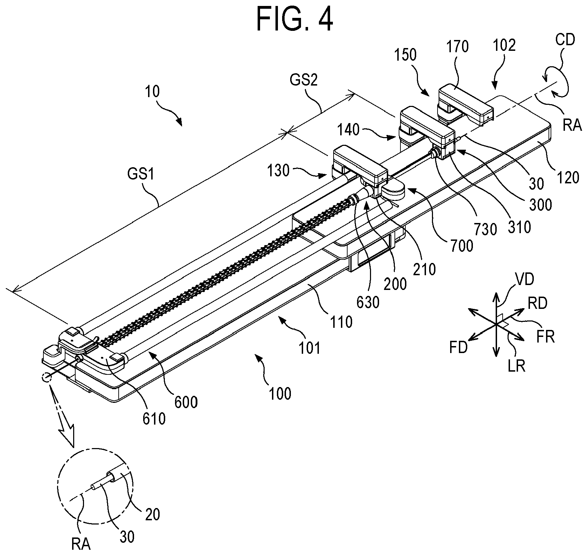

In the present disclosure, a frontward direction means a direction in which a longitudinal side of a platform of a vascular intervention procedure device is directed toward a patient (a direction indicated by a symbol FD in FIG. 4), a rearward direction means a direction opposite to the frontward direction, and a front and rear direction includes the frontward direction and the rearward direction. In the present disclosure, a transverse direction means a direction orthogonal to the front and rear direction, i.e., a direction in which a short side of the platform is positioned, and includes a leftward direction and a rightward direction. In the present disclosure, a vertical direction means a direction perpendicular to both the front and rear direction and the transverse direction.

Hereinafter, the embodiments are described with reference to the accompanying drawings. Like reference numerals in the accompanying drawings denote like or corresponding elements. Further, in the following description of the embodiments, redundant descriptions for the same or corresponding elements may be omitted. However, even if the descriptions of the elements are omitted, such elements are not intended to be excluded in any embodiment.

The embodiments disclosed hereinafter and the embodiments shown in the accompanying drawings relate to a vascular intervention procedure device which is used for transferring and rotating procedure tools to be used in a vascular intervention procedure, and for introducing the procedure tools into a target blood vessel. The vascular intervention procedure device according to the embodiments is used for a vascular intervention procedure where a catheter, a guide wire, a micro catheter, and a micro guide wire are used. In the present disclosure, the catheter, the guide wire, the micro catheter, and the micro guide wire are referred to as a procedure tool.

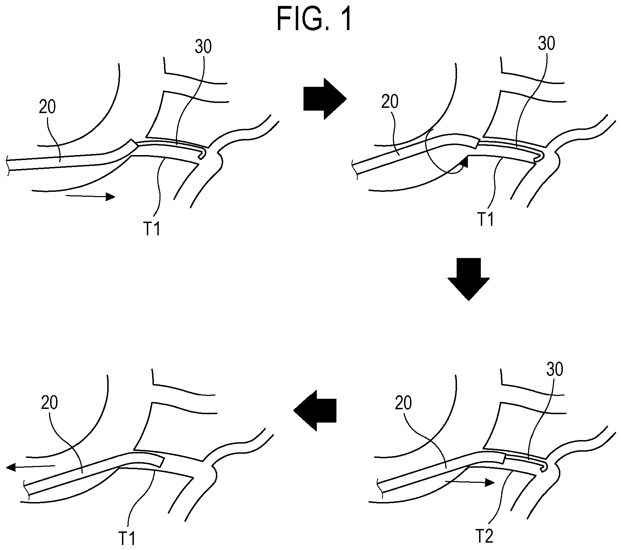

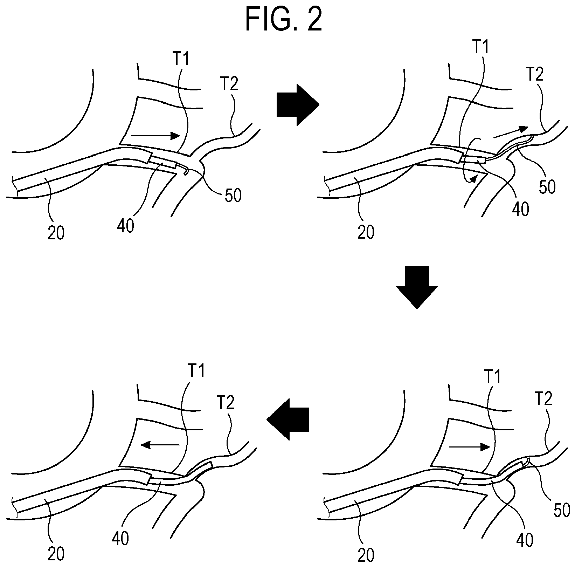

The catheter is a flexible tube, and is introduced into a target blood vessel. The guide wire is inserted into the catheter in order to guide the catheter to the target blood vessel. The micro catheter can be inserted into the catheter, and is a flexible tube. The micro catheter is introduced into a narrower target blood vessel into which the catheter cannot be introduced, and is used for injecting medicinal fluid into the narrower target blood vessel or suctioning a blood clot. The micro guide wire has a thickness smaller that the guide wire, and is used for guiding the micro catheter to the narrower target blood vessel. FIGS. 1 and 2 schematically show examples of insertion and rotation of the procedure tools in the vascular intervention procedure.

An example where a catheter reaches the target blood vessel by the vascular intervention procedure device by using the catheter and the guide wire is described with reference to FIG. 1. To introduce a catheter 20 and a guide wire 30 into the vicinity of the target blood vessel, the vascular intervention procedure device can transfer the catheter 20 and the guide wire 30. To introduce the guide wire 30 into a first target blood vessel T1, the vascular intervention procedure device can transfer and rotate the guide wire 30. Further, the vascular intervention procedure device can rotate the catheter 20 together with the transfer and rotation of the guide wire 30. If the guide wire 30 is introduced into the first target blood vessel T1, the vascular intervention procedure device can introduce the catheter 20 into the first target blood vessel T1 along the guide wire 30. If the catheter 20 reaches the first target blood vessel T1, the guide wire 30 is removed from the catheter 20. The catheter 20 can be used for injecting medicinal fluid into the first target blood vessel T1, or suctioning a blood clot in the first target blood vessel.

An example where the micro catheter reaches the target blood vessel by the vascular intervention procedure device by using the catheter, the micro catheter, and the micro guide wire is described with reference to FIG. 2. In the state where the catheter 20 reaches the first target blood vessel T1, injection of a medicinal fluid or suction of a blood clot may be needed for a second target blood vessel T1 narrower than the first target blood vessel T1. A micro catheter 40 is inserted into the catheter 20, and a micro guide wire 50 is inserted into the micro catheter 40. The vascular intervention procedure device introduces the micro guide wire 50 into the second target blood vessel T2 by transferring and rotating the micro guide wire 50. Thereafter, the vascular intervention procedure device transfers the micro catheter 40 in order to introduce the micro catheter 40 into the second target blood vessel T2 along the micro guide wire 50. If the micro catheter 40 reaches the second target blood vessel T2, the micro guide wire 50 is removed from the micro catheter 40. The micro catheter 40 can be used for injecting medicinal fluid into the second target blood vessel T2, or suctioning a blood clot. Alternatively, for purposes of additional procedure, various devices can be introduced into the second target blood vessel through the micro catheter 40.

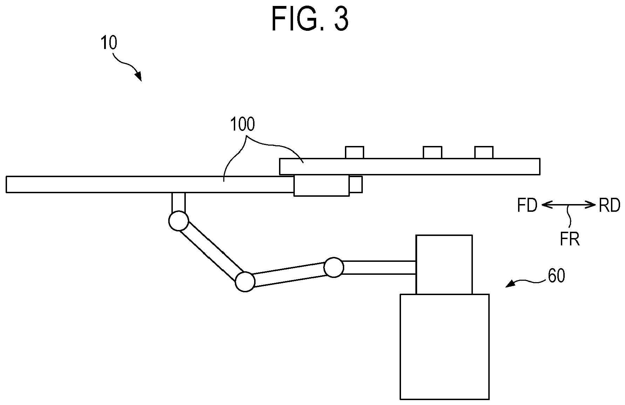

FIG. 3 schematically shows an example of a state where the vascular intervention procedure device according to one embodiment is used for the vascular intervention procedure. The vascular intervention procedure device 10 includes a platform 100 for transferring and rotating the above-described procedure tools. The platform 100 of the vascular intervention procedure device 10 may be removably mounted on an arm of a multi-degree-of-freedom stand device 60. Therefore, a clinician easily moves the vascular intervention procedure device 10 to a desired position, and can position a front end of the platform 100 such that the front end of the platform is directed toward a procedure side of a patient. The multi-degree-of-freedom stand device 60 may be located in the vicinity of an operating table on which the patient lies. By way of other example, a mechanism, which can support, rotate and move the vascular intervention procedure device 10, may be provided in the aforementioned operating table, and the vascular intervention procedure device 10 may be removably mounted on such a mechanism.

Hereinafter, the embodiments of the vascular intervention procedure device are described with reference to FIGS. 4 to 38.

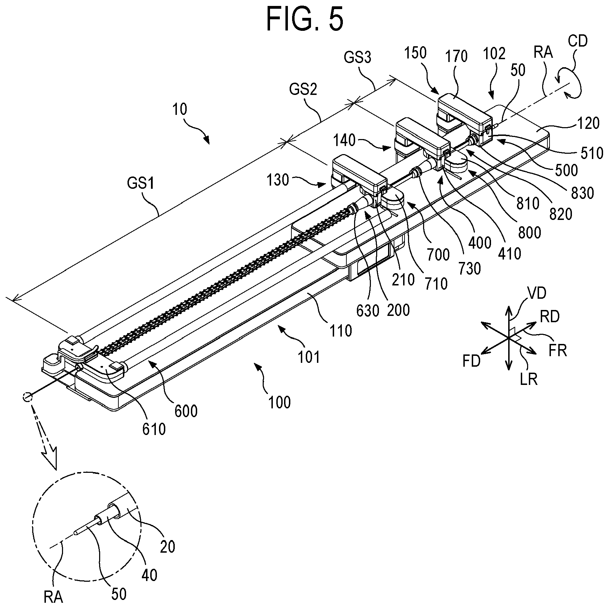

FIG. 4 is a perspective view showing the vascular intervention procedure device according to one embodiment in one operation mode, and FIG. 5 is a perspective view showing the vascular intervention procedure device according to one embodiment in another operation mode.

The vascular intervention procedure device 10 shown in FIG. 4 can be operated for the vascular intervention procedure using the catheter 20 and the guide wire 30 (e.g., the vascular intervention procedure illustrated in FIG. 1), and the operation mode shown in FIG. 4 is referred to as a first operation mode. The vascular intervention procedure device 10 shown in FIG. 5 can be operated for the vascular intervention procedure, which uses the micro catheter 40 and the micro guide wire 50 in a state where the catheter 20 reaches a target blood vessel (e.g., the vascular intervention procedure illustrated in FIG. 2), and the operation mode shown in FIG. 5 is referred to as a second operation mode. The vascular intervention procedure device 10 according to one embodiment may be configured to have the first operation mode of transferring or rotating the catheter 20 and the guide wire 30 independently, and the second operation mode of transferring or rotating the catheter 20, the micro catheter 40, and the micro guide wire 50 independently.

In the first operation mode shown in FIG. 4, a catheter module 200 supporting the catheter 20, and a guide wire module 300 supporting the guide wire 30 are coupled to the platform 100 of the vascular intervention procedure device 10. In the second operation mode shown in FIG. 5, the catheter module 200 supporting the catheter 20, a micro catheter module 400 supporting the micro catheter 40, and a micro guide wire module 500 supporting the micro guide wire 50 are coupled to the platform 100 of the vascular intervention procedure device 10.

In the present disclosure, the procedure tool, which is first disposed in a direction directed from the front end of the platform 100 toward a rear end thereof, is referred to as a first procedure tool, and the procedure tool, which is inserted into the first procedure tool in rear of the first procedure tool, is referred to as a second procedure tool. The procedure tool inserted into the second procedure tool in rear of the second procedure tool is referred to as a third procedure tool. In FIG. 4, the first procedure tool is the catheter 20, and the second procedure tool is the guide wire 30. In FIG. 5, the first procedure tool is the catheter 20, the second procedure tool is the micro catheter 40, and the third procedure tool is the micro guide wire 50.

Further, in the present disclosure, the modules, which support the aforementioned procedure tools respectively and are coupled to the platform 100, are referred to as procedure tool modules. The procedure tool module, which is first disposed in the direction directed from the front end of the platform 100 toward the rear end thereof, is referred to as a first procedure tool module, and the procedure tool module, which is disposed in rear of the first procedure tool module, is referred to as a second procedure tool. The procedure tool module disposed in rear of the second procedure tool module is referred to as a third procedure tool module. In FIG. 4, the first procedure tool module is the catheter module 200, and the second procedure tool module is the guide wire module 300. In FIG. 5, the first procedure tool module is the catheter module 200, the second procedure tool module is the micro catheter module 400, and the third procedure tool module is the micro guide wire module 500.

Referring to FIG. 4, the vascular intervention procedure device 10 according to one embodiment includes the platform 100, the catheter module 200, and the guide wire module 300. The platform 100 is a base of the vascular intervention procedure device 10, and generates power for transferring and rotating the procedure tools. The catheter module 200 (the first procedure tool module) and the guide wire module 300 (the second procedure tool module) are disposed on the platform 100. The catheter module 200 is configured to support and rotate the catheter 20 (the first procedure tool). The guide wire module 300 is configured to support and rotate the guide wire 30 (the second procedure tool).

Referring to FIG. 5, the vascular intervention procedure device 10 according to one embodiment includes the platform 100, the catheter module 200, the micro catheter module 400, and the micro guide wire module 500. The catheter module 200 (the first procedure tool module), the micro catheter module 400 (the second procedure tool module), and the micro guide wire module 500 (the third procedure tool module) are disposed on the platform 100. The micro catheter module 400 is configured to support the micro catheter 40 (the second procedure tool). The micro guide wire module 500 is configured to support and rotate the micro guide wire 50 (the third procedure tool).

In the vascular intervention procedure device 10 shown in FIG. 5, the micro catheter module 400 is employed as the second procedure tool module by replacing the guide wire module shown in FIG. 4. The micro catheter module 400 which is the second procedure tool module is not used together with the guide wire module, and can be employed in the vascular intervention procedure device 10 by replacing the guide wire module.

Referring to FIGS. 4 and 5, the platform 100 is positioned in a front and rear direction FR. The platform 100 may be positioned in the front and rear direction FR as supported by the multi-degree-of-freedom stand device shown in FIG. 3.

The platform 100 may be configured to transfer at least one of the aforementioned procedure tools in the front and rear direction, and to generate rotation power for rotating the same about a rotation axis of the front and rear direction. The platform 100 may be comprised of a single module, or may be comprised of two modules that are relatively movably coupled to each other. The platform 100 may include at least one transferring portion for transferring and rotating at least one of the aforementioned procedure tools. Such a transferring portion may be disposed in the aforementioned single module, or a movable module of the aforementioned two modules.

The platform 100 of one embodiment may be comprised of a fixed base module 101 and a movable transfer module 102. The base module 101 is located below, and the transfer module 102 is located above a base frame 110. The base module 101 includes the base frame 110 extending in the front and rear direction. The base frame 110 may be separably fixed to the aforementioned multi-degree-of-freedom stand device. The transfer module 102 may be moved with respect to the base frame 110 in the front and rear direction FR. The transfer module 102 includes a transfer frame 120 which is coupled to the base frame 110 so as to be transferred in the front and rear direction FR along the base frame 110.

The catheter module 200 and the guide wire module 300 shown in FIG. 4 are disposed on the transfer module 102. The catheter module 200, the micro catheter module 400, and the micro guide wire module 500 shown in FIG. 5 are disposed on the transfer module 102. The transfer module 102 is configured to transfer the above-described procedure tool modules. The aforementioned procedure tool modules are coupled to the transfer module 102, and can be transferred independently or simultaneously in the front and rear direction FR by the transfer module 102.

Referring to FIG. 4, the catheter module 200 supporting the catheter 20 (the first procedure tool) is coupled to the transfer module 102. The catheter module 200 is transferred with respect to the base frame 110 in the front and rear direction FR. The transfer module 102 is transferred in a frontward direction FD along the base frame 110, whereby the catheter 20 (the first procedure tool) of the catheter module 200 can be transferred frontward. Therefore, the catheter 20 can be introduced into the target blood vessel along the guide wire 30. The guide wire module 300 (the second procedure tool module) supporting the guide wire 30 (the second procedure tool) is coupled to the transfer module 102 in rear of the catheter module 200. Further, the transfer module 102 is configured to transfer the guide wire module 300 in the front and rear direction FR independently of the catheter module 200. The guide wire module 300 is transferred with respect to the base frame 110 in the front and rear direction FR. The transfer module 102 is transferred in the frontward direction FD along the base frame 110, whereby the guide wire 30 can be transferred frontward. Further, the guide wire 30 of the guide wire module 300 can be transferred frontward by a transferring portion 140 of the transfer module 102. Therefore, the guide wire 30 (the second procedure tool) inserted into the catheter 20 (the first procedure tool) can be introduced into the target blood vessel. FIG. 4 shows that the guide wire module 300 is coupled to the transferring portion 140, but the guide wire module 300 may be coupled to a transferring portion 150 in other embodiment.

Referring to FIG. 5, the catheter module 200 supporting the catheter 20 (the first procedure tool) is coupled to the transfer module 102, and the catheter module 200 is transferred with respect to the base frame 110 in the front and rear direction FR. The micro catheter module 400 (the second procedural module) supporting the micro catheter 40 (the second procedure tool) is coupled to the transfer module 102 in rear of the catheter module 200. The transfer module 102 is configured to transfer the micro catheter module 400 in the front and rear direction FR independently of the catheter module 200. The micro catheter module 400 is transferred with respect to the base frame 110 in the front and rear direction FR. The transfer module 102 is transferred in the frontward direction FD along the base frame 110, whereby the micro catheter 40 can be transferred frontward. Further, the micro catheter 40 of the micro catheter module 400 can be transferred by the transferring portion 140 of the transfer module 102. Therefore, the micro catheter 40 (the second procedure tool) inserted into the catheter 20 (the first procedure tool) can be introduced into the target blood vessel.

The micro guide wire module 500 supporting the micro guide wire 50 (the third procedure tool) is coupled to the transfer module 102 in rear of the micro catheter module 400. The transfer module 102 is configured to transfer the micro guide wire module 500 in the front and rear direction FR independently of the catheter module 200 and the micro catheter module 400. The micro guide wire module 500 is transferred with respect to the base frame 110 in the front and rear direction FR by the transfer module 102. The transfer module 102 is transferred in the frontward direction FD along the base frame 110. Further, independently of the catheter (the first procedure tool) of the catheter module 200 or the micro catheter (the second procedure tool) of the micro catheter module 400, the micro guide wire (the third procedure tool) of the micro guide wire module 500 is transferred frontward by the transfer module 102. Therefore, the micro guide wire inserted in the micro catheter 40 can be introduced into the target blood vessel.

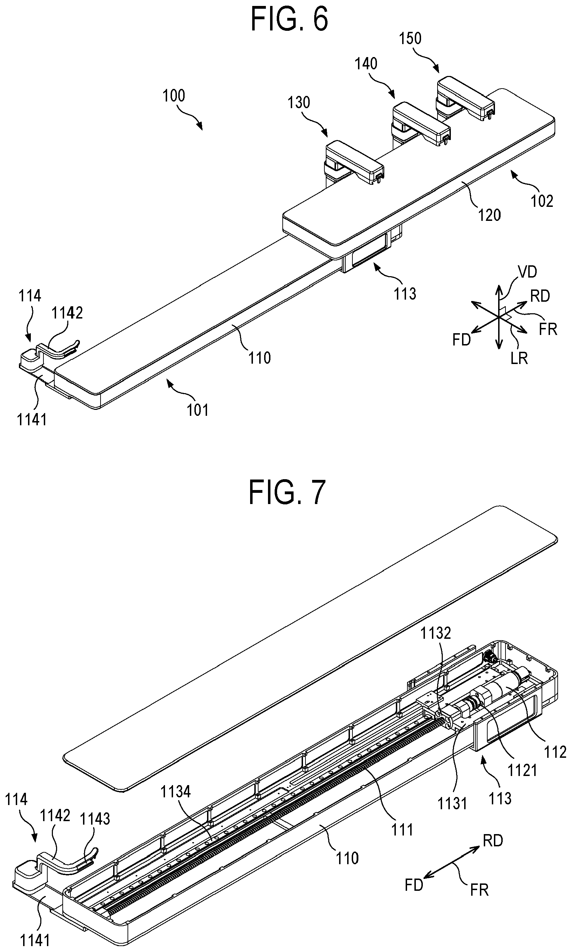

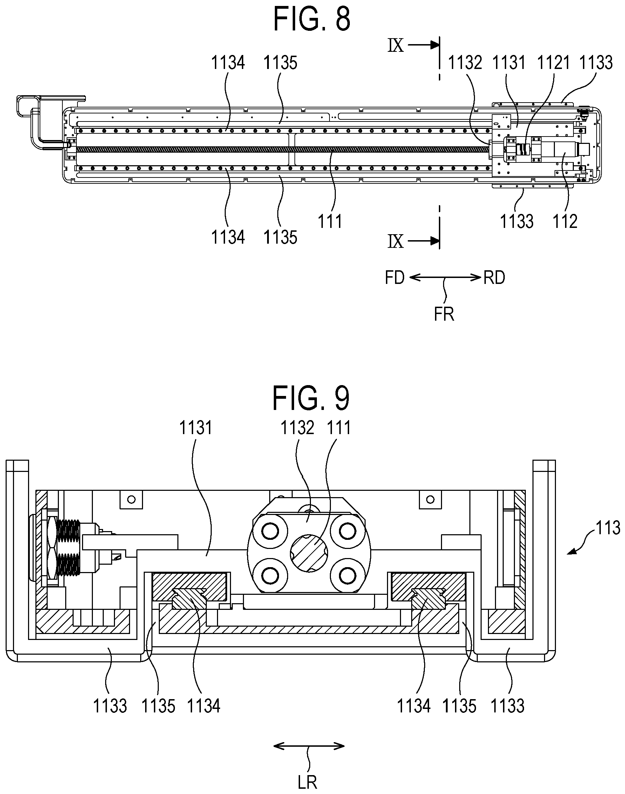

FIG. 6 is a perspective view showing the platform of the vascular intervention procedure device according to one embodiment. FIG. 7 is a perspective view showing the base frame of the platform shown in FIG. 6. FIG. 8 is a plan view showing the base frame of the platform shown in FIG. 6. FIG. 9 is a cross-sectional view taken along line IX-IX of FIG. 8. Hereinafter, reference is made to FIGS. 6 to 9.

The base frame 110 may be formed as a housing including drive components therein. The transfer frame 120 of the transfer module 102 is coupled to the base frame 110 so as to be slidable in the front and rear direction FR.

The vascular intervention procedure device includes a transfer module driving portion transferring the transfer module 102 with respect to the base frame 110. The transfer module driving portion may be disposed in the base frame 110 or in the transfer frame 120. According to one embodiment, the transfer module driving portion is disposed in the inside of the base frame 110. The transfer frame 120 may be slidably coupled to the base frame 110 by the transfer module driving portion.

The transfer module driving portion includes a frame transferring lead screw 111, a frame transferring motor 112 for rotating the frame transferring lead screw 111, and a frame slider 113 coupled to the frame transferring lead screw 111 and the transfer frame 120 and transferred along the frame transferring lead screw 111 through screw motion. The frame transferring lead screw 111 is disposed on a bottom portion of the base frame 110 along the front and rear direction FR, and is supported rotatably. The frame transferring motor 112 is connected to the frame transferring lead screw 111 through a coupling 1121. The frame transferring motor 112 rotates the frame transferring lead screw 111 in a clockwise direction or in a counterclockwise direction. Therefore, the transfer module 102 coupled to the frame slider 113 is transferred in the frontward direction FD or in the rearward direction RD.

The frame slider 113 is configured to support the transfer frame 120 with respect to the base frame 110, and transfers the transfer frame 120 by the rotation of the frame transferring lead screw. The frame slider 113 includes a slide portion 1131 having a transferring nut 1132, which is coupled to the frame transferring lead screw 111 so as to be transferred through screw motion, and includes a pair of supporting portions 1133 extending from the slide portion 1131 in a transverse direction LR respectively and coupled to a bottom portion of the transfer frame 120. A pair of linear rails 1134 are disposed on an upper surface of the bottom portion of the base frame 110 in the front and rear direction FR. Slits 1135 are perforated through the bottom portion of the base frame 110, and are formed along each of the linear rails 1134. The slide portion 1131 is slidably coupled to the linear rails 1134. Each supporting portion 1133 extends upward through the slit 1135. Each supporting portion 1133 is formed so as to space an upper surface of the base frame 110 and a lower surface of the transfer frame 120 in a vertical direction. The transfer frame 120 coupled to the frame slider 113 is connected to the base frame 110 with a gap in the vertical direction therebetween. Since the transfer frame 120 is supported and transferred by the frame slide 113, the transfer frame 120 can be coupled to the base frame 110 without friction between the transfer frame and the base frame 110.

Further, the base frame 110 includes, at its front end (the front end of the platform), a guide module holder 114. The guide module holder 114 may be separably coupled to a guide housing of a first guide module to be described below. The guide module holder 114 includes a stand 1141 protruding from a front end of the base frame 110 in the transverse direction, and a housing holder 1142 coupled to the stand 1141 so as to be separable in the vertical direction. A fitting groove 1143 may be formed in the housing holder 1142 in the front and rear direction.

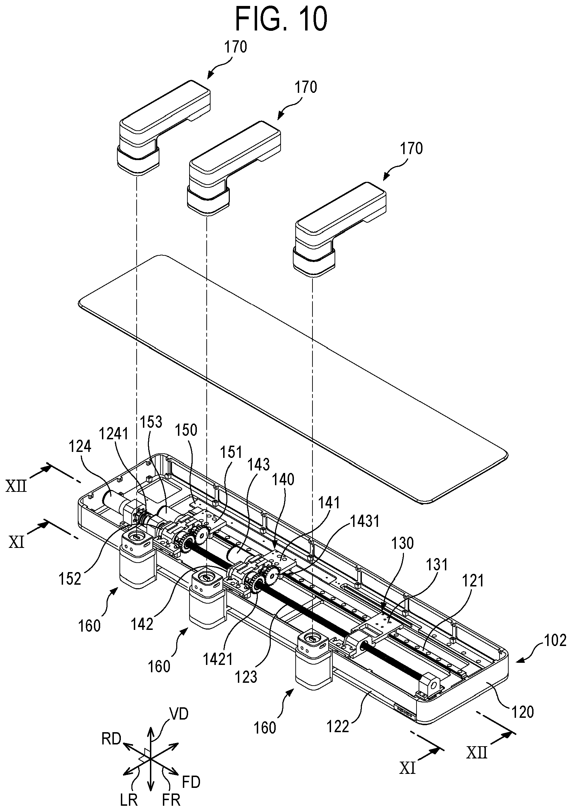

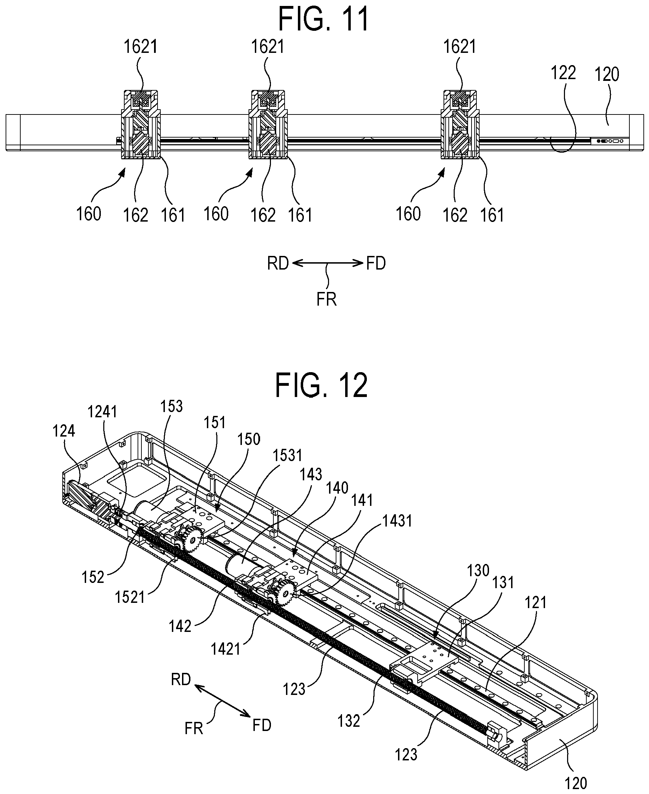

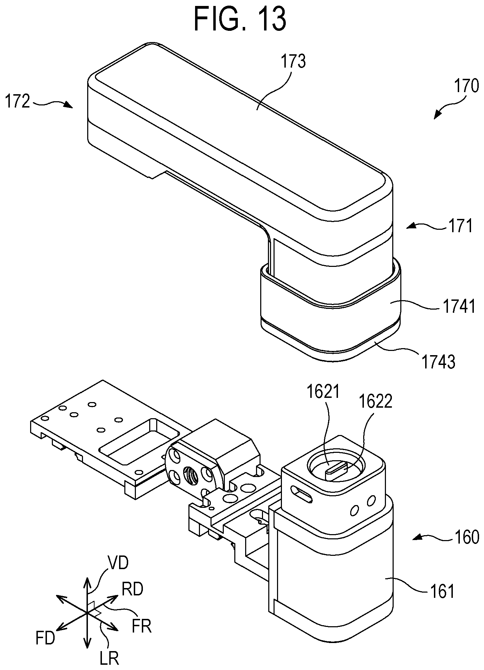

FIG. 10 is an exploded perspective view showing the transfer frame of the platform shown in FIG. 6. FIG. 11 is a cross-sectional view taken along line XI-XI of FIG. 10. FIG. 12 is a cross-sectional view taken along line XII-XII of FIG. 10. FIG. 13 is a perspective view showing an example of the transferring portion shown in FIG. 10. Hereinafter, reference is made to FIGS. 4 to 6 and 10 to 13.

The transfer module 102 of one embodiment includes the transferring portions 130, 140 and 150 transferring the procedure tool modules. The catheter module 200 is coupled to the transferring portion 130. The guide wire module 300 is coupled to the transferring portion 140 or the transferring portion 150. The micro catheter module 400 is coupled to the transferring portion 140. The micro guide wire module 500 is coupled to to the transferring portion 150. The transfer module 102 may be configured to simultaneously transfer the transferring portion 130, the transferring portion 140, and the transferring portion 150 in the front and rear direction. The transfer module 102 may be configured such that the transferring portion 140 is transferred independently of the transferring portion 130. The transfer module 102 may be configured such that the transferring portion 150 is transferred independently of the transferring portion 130 and the transferring portion 140.

The transferring portion 130 and the transferring portion 140 may be disposed in the transfer frame 120 so as to be transferable in the front and rear direction FR. The transferring portion 140 is disposed in the transfer frame 120 in rear of the transferring portion 130. The transferring portion 150 may be disposed in the transfer frame 120 so as to be transferable in the front and rear direction FR. The transferring portion 150 is disposed in the transfer frame 120 in rear of the transferring portion 130. Alternatively, the transferring portion 150 is disposed in the transfer frame 120 in rear of the transferring portion 140.

The transfer frame 120 may be formed as a housing including drive components therein. The frame slider 113 *(see* FIG. 6) of the base frame may be separably coupled to the lower surface of the transfer frame 120. FIG. 10 shows the drive components in the transfer frame 120, which is seen by removing an upper portion of the transfer frame 120.

A pair of linear rails 121 extending in the front and rear direction FR are disposed in the upper surface of the bottom portion of the transfer frame 120, and a slit 122 is formed through a lateral wall portion of the transfer frame 120 in the front and rear direction FR. The plurality of transferring portions 130, 140, and 150 may have the same configuration. The transferring portions 130, 140, and 150 may have a module sliders 131, 141, and 151, respectively which are slidably coupled to the linear rail 121. Each of the transferring portions 130, 140 and 150 may be slidably transferred in the front and rear direction FR along the linear rail 121. Each of the module sliders 131, 141, and 151 extends in the transverse direction LR orthogonal to the front and rear direction FR, and its end portion may protrude through the slit 122 outward from the transfer frame 120.

The transfer module 102 may transfer the plurality of transferring portions 130, 140, and 150 simultaneously in the front and rear direction. The transfer module 102 may include a module transferring lead screw 123, which is rotatably supported on the bottom portion of the transfer frame 120 and is disposed along the front and rear direction FR. The transfer module 102 may include a module transferring motor 124 for rotating the module transferring lead screw 123. The module transferring motor 124 is connected to the module transferring motor 124 through a coupling 1241. The module transferring motor 124 rotates the module transferring lead screw 123 in a clockwise direction or in a counterclockwise direction.

The module transferring lead screw 123 is coupled to each of the transferring portions 130, 140, and 150 so as to transfer each of the transferring portions 130, 140, and 150 through screw motion. The module transferring lead screw 123 may be coupled to the module slider 131, 141, and 151 of each of the transferring portions.

The transferring portion 130 includes a transferring nut 132 (a first transferring nut), which is coupled to the module transferring lead screw 123 so as to be transferred through screw motion. The transferring nut 132 is disposed in the module slider 131. The transferring portion 130 is transferred in the front and rear direction FR by the rotation of the module transferring lead screw 123.

The transferring portion 140 is disposed so as to be transferred in the front and rear direction FR independently of the transferring portion 130. The transferring portion 140 includes a transferring nut 142 (a second transferring nut), which is coupled to the module transferring lead screw 123 so as to be transferred through screw motion. The transferring nut 142 is disposed in the module slider 141. The transferring portion 140 is transferred in the front and rear direction FR by the rotation of the module transferring lead screw 123.

Further, the transferring portion 140 may include a transferring motor 143 (a second transferring motor) which is configured to rotate the transferring nut 142 *(see* FIG. 10 and FIG. 12). The transferring motor 143 may be disposed in the module slider 141. A rotary shaft of the transferring motor 143 and the transferring nut 142 may be interconnected through gear power transmission. By way of example, a spur gear 1431 is coupled to the rotary shaft of the transferring motor 143, and a spur gear 1421 meshing with the spur gear 1431 may be integrally coupled to the transferring nut 142. However, the rotary shaft of the transferring motor 143 and the transferring nut 142 may be interconnected through other structure such as a bevel gear or the like. If the transferring nut 142 is rotated, the transferring portion 140 is transferred in the front and rear direction FR along the module transferring lead screw 123. Since the transferring nut 142 can be rotated by the transferring motor 143 independently of the rotation of the module transferring lead screw 123, the transferring portion 140 can be transferred in the front and rear direction independently of the transferring portion 130.

The transferring portion 150 is disposed so as to be transferred in the front and rear direction FR independently of the transferring portion 130 and the transferring portion 140. The transferring portion 150 includes a transferring nut 152 (a third transferring nut), which is coupled to the module transferring lead screw 123 through screw motion. The transferring nut 152 is disposed in the module slider 151. The transferring portion 150 is transferred in the front and rear direction FR by the rotation of the module transferring lead screw 123.

Further, the transferring portion 150 may include a transferring motor 153 (a third transferring motor) configured to rotate the transferring nut 152 *(see* FIG. 10 and FIG. 12). The transferring motor 153 may be disposed in the module slider 151. A rotary shaft of the transferring motor 153 and the transferring nut 152 may be interconnected through gear power transmission. By way of example, a spur gear 1531 is coupled to the rotary shaft of the transferring motor 153, and a spur gear 1521 meshing with the spur gear 1531 may be integrally coupled to the transferring nut 152. However, the rotary shaft of the transferring motor 153 and the transferring nut 152 may be interconnected through other structure such as a bevel gear or the like. If the transferring nut 152 is rotated, the transferring portion 150 is transferred in the front and rear direction FR along the module transferring lead screw 123. Since the transferring nut 152 can be rotated by the transferring motor 153 independently of the rotation of the module transferring lead screw 123, the transferring portion 150 can be transferred in the front and rear direction independently of the transferring portion 130 and the transferring portion 140.

In the vascular intervention procedure device of another embodiment which is not shown, the transferring portion 130 may be configured to be fixed to the transfer module 102. In such an example, as the transfer frame 120 is transferred in the front and rear direction FR along the base frame 110, the transferring portion 130 can be transferred in the front and rear direction FR. Further, in such an example, the module transferring lead screw 123 may be disposed in the transfer frame 120 so as to move the transferring portions 140 and 150 through screw motion, and the transferring portion 140 may be configured without the transferring motor 143.

In the vascular intervention procedure device of another embodiment which is not shown, the transfer module 102 may include only the transferring portion 130 and the transferring portion 140. In such an example, only the catheter module 200 and the guide wire module 300 may be coupled to the transferring portion 130 and the transferring portion 140 respectively, and the vascular intervention procedure device may be used for the vascular intervention procedure illustrated in FIG. 1.

As another embodiment, the platform 100 may include a single, modularized frame (e.g., the base frame). In such an example, the above-described transferring portions may be disposed in the aforementioned single frame.

Referring to FIG. 4 and 5, the catheter 20 of the catheter module 200 can be transferred in the front and rear direction FR by transfer of the transfer frame 120 or transfer of the transferring portion 130. The catheter module 200 is configured to rotate the catheter 20 about a rotation axis RA of the front and rear direction FR.

The guide wire 30 of the guide wire module 300 can be transferred in the front and rear direction FR by the transfer of the transfer frame 120 or the transfer of the transferring portion 140 or the transferring portion 150. The guide wire module 300 is configured to rotate the guide wire 30 about the rotation axis RA.

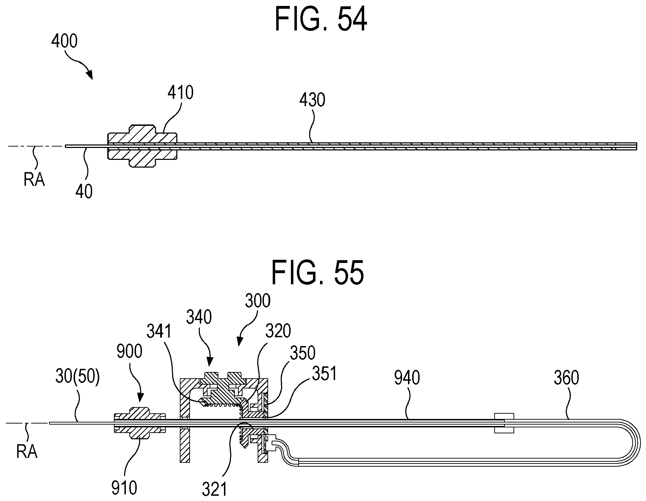

The micro catheter 40 of the micro catheter module 400 can be transferred in the front and rear direction FR by the transfer of the transfer frame 120 or the transfer of the transferring portion 140. The micro catheter module 400 is configured to support the micro catheter 40. The micro catheter module 400 may be configured to fix the micro catheter 40 such that the micro catheter cannot be rotated, or may be configured to rotatably support the micro catheter 40 *(see* an embodiment according to FIG. 54 to be described below).

The micro guide wire 50 of the micro guide wire module 500 can be transferred in the front and rear direction FR by the transfer of the transfer frame 120 or the transfer of the transferring portion 150. The micro guide wire module 500 is configured to rotate the micro guide wire 50 about the rotation axis RA.

The transfer module 102 may be configured to generate rotation power for rotating the procedure tool in each of the procedure tool modules and to transmit the rotation power to each module. Each transferring portion of the transfer frame 120 may be configured to generate and transmit the aforementioned rotation power. The transferring portion 130 is configured to generate rotation power for rotating the catheter 20 and to transmit the rotation power to the catheter module 200, in the state of being coupled to the catheter module 200. The transferring portion 140 is configured to generate rotation power for rotating the second procedure tool (the guide wire or the micro catheter) and to transmit the rotation power to the second procedure tool module. Where the second procedure tool module is the guide wire module 300, the transferring portion 140 is configured to generate the rotation power for rotating the guide wire 30 and to transmit the rotation power to the guide wire module 300, in the state of being coupled to the guide wire module 300. Where the second procedure tool module is the micro catheter module 400, the transferring portion 140 is configured to generate and transmit the rotation power, but the micro catheter module 400 may be operated so as not to rotate the micro catheter. The transferring portion 150 is configured to generate rotation power for rotating the micro guide wire 50 and to transmit the rotation power to the micro guide wire module 500, in the state of being coupled to the micro guide wire module 500.

In another embodiment, each of the transferring portions is configured to only transmit the rotation power, and a rotation power generating portion may be disposed in the transfer frame so as to generate the rotation power that each transferring portion transmits.

In the embodiment of the vascular intervention procedure device, at least any one of the transferring portions of the platform includes a rotation power generating portion generating the rotation power, and a power transmitting portion configured to transmit the rotation power to any one of the procedure tool modules. The power transmitting portion is configured to be separably coupled to the rotation power generating portion. Further, the power transmitting portion is configured to be separably coupled to any one of the procedure tool modules. Accordingly, since the procedure tool module supporting and rotating the procedure tool, and the transferring portion of the platform are connected through the power transmitting portion, the procedure tool modules and the platform can have a simplified structure.

The rotation power generating portion may generate the rotation power that rotates any corresponding procedure tool among the catheter, the guide wire, and the micro guide wire. Further, the power transmitting portion may transmit the rotation power to any corresponding procedure tool module among the catheter module, the guide wire module, and the micro guide wire module. In one embodiment of the vascular intervention procedure device, each of the transferring portions may include the rotation power generating portion and the power transmitting portion.

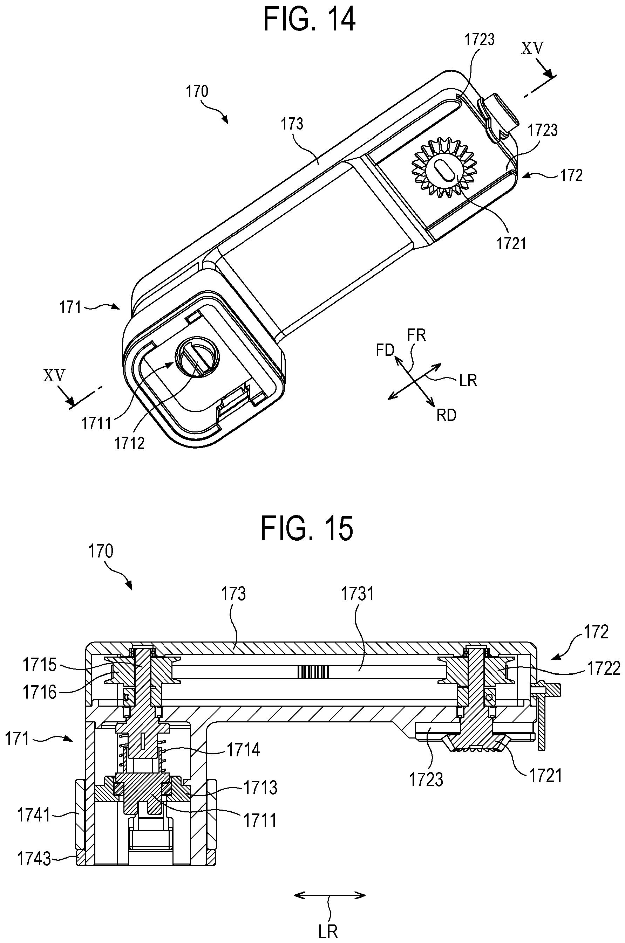

FIGS. 14 to 17 show the components of the transferring portion shown in FIG. 13. Hereinafter, reference is made to FIGS. 4, 5, 10, 11, and 13 to 17 with regard to the transferring portion of one embodiment.

Each of the transferring portions 130, 140, and 150 may include a rotation power generating portion 160 generating rotation power. Each of the transferring portions 130, 140, and 150 may include a power transmitting portion 170 transmitting the rotation power.

The rotation power generating portion 160 of the transferring portion 130 may be connected to a portion of the module slider 131, which protrudes through the slit 122 toward a lateral side of the transfer frame 120. The rotation power generating portion 160 includes a motor housing 161 coupled to the module slider 131, a rotary motor 162 disposed in the motor housing 161, and a first rotator 1621 coupled to a rotary shaft of the rotary motor 162 and rotatable so as to output the rotation power of the rotary motor 162. The rotation power generating portions 160 of the transferring portions 140 and 150 may have the same structure as the structure of the rotation power generating portion 160 of the transferring portion 130. The rotation power generating portions 160 of the transferring portions 140 and 150 may be connected to a portion of each of the module sliders 141 and 151, which protrude through the slit 122, respectively. In another example which is not shown, the rotation power generating portions may be disposed inside of the transfer frame 120.

The power transmitting portion 170 may be disposed at the outside of the transfer frame 120. The power transmitting portion 170 of the transferring portion 140 may be separably coupled to the rotation power generating portion 160 in a vertical direction VD. The power transmitting portion 170 of the transferring portion 140 is coupled to the catheter module 200. The power transmitting portion 170 of the transferring portion 140 is configured to receive the rotation power from the rotation power generating portion 160 and to transmit the same to the catheter module 200.

Since the rotation power generating portion 160 and the power transmitting portion 170 are disposed at the outside of the transfer frame 120 (e.g., at a lateral side of the transfer frame 120), the transferring portion 130 is configured to transmit the rotation power to the catheter module 200 in the transverse direction LR.

The power transmitting portion 170 may have a shape that is bent so as to correspond to the exterior shape of the transfer frame 120 (or the platform 100). As shown in FIG. 4, when the transfer module 102 (the platform 100) is viewed in the front and rear direction FR, the power transmitting portion 170 may be formed to extend in a circumferential direction CD centered on the rotation axis RA. For example, the power transmitting portion 170 may have an inverted L-shape so as to correspond to an exterior shape of a lateral wall portion and a top portion of the transfer frame.

The power transmitting portions 170 of the transferring portions 140 and 150 may have the same structure as the structure of the power transmitting portion 170 of the transferring portion 130. The power transmitting portion 170 of the transferring portion 140 may transmit the rotation power of the rotation power generating portion 160 to the second procedure tool module (the guide wire module 300 or the micro catheter module 400). The power transmitting portion 170 of the transferring portion 150 may transmit the rotation power of the transferring portion 150 to the micro guide wire module 500.

According to the embodiments, the rotation power generating portion and the power transmitting portion of each transferring portion may be separably coupled to each other through fitting. Further, the procedure tool module supporting and rotating the procedure tool is separably coupled to the power transmitting portion of the transferring portion. The power transmitting portion of each transferring portion, and the procedure tool module corresponding to each transferring portion may be separably coupled to each other through fitting. Therefore, in the vascular intervention procedure device of the embodiments, each of the functional components can be modularized, each of the functional components can be employed in the platform selectively according to necessity, and usability and convenience can be enhanced.

According to the embodiments, each of the power transmitting portions may be configured to be coupled to the procedure tool module corresponding thereto in the transverse direction orthogonal to the front and rear direction through fitting. By way of another example, each of the power transmitting portions and the procedure tool module corresponding thereto may be configured to be coupled to each other through fitting in the front and rear direction or in an oblique direction between the front and rear direction and the transverse direction.

Hereinafter, the rotation power generating portion and the power transmitting portion are described in detail with reference to FIGS. 13 to 17.

The power transmitting portion 170 includes an input end portion 171 receiving the rotation power from the rotation power generating portion, and an output end portion 172 outputting the rotation power to the corresponding procedure tool module. The power transmitting portion 170 includes a bridge portion 173 interconnecting the input end portion 171 and the output end portion 172. The input end portion 171 may be formed such that the output end portion of the rotation power generating portion 160 is fitted into the input end portion in the vertical direction. The input end portion 171 may be located at the lateral side of the transfer frame. The output end portion 172 may be separably coupled to the corresponding procedure tool module. The output end portion 172 may be located above the transfer frame (above the platform). The procedure tool modules may be disposed between the transfer frame and the output end portion 172 in the state of being coupled to the output end portions of the power transmitting portions, respectively.

The rotation power generating portion 160 includes, on its upper end portion, the first rotator 1621 outputting the rotation power. The power transmitting portion 170 includes, in the output end portion 172, a second rotator 1711 corresponding to the first rotator 1621. The second rotator 1711 is configured to have a shape complementary to the first rotator 1621. One of the first rotator 1621 and the second rotator 1711 may have a protruding portion, and the other of the first rotator 1621 and the second rotator 1711 may have a concave portion to which the protruding portion is fitted. By way of example, as shown in FIG. 13, the first rotator 1621 has a protruding portion 1622 protruding upward and, as shown in FIG. 14, the second rotator 1711 has a concave portion 1712 to which the protruding portion 1622 can be fitted.

The power transmitting portion 170 may include, in the input end portion 171, a rotator guide 1713 which movably maintains the second rotator 1711 in the vertical direction, in order that the power transmitting portion 170 can receive the rotation power while the rotation power generating portion 160 and the power transmitting portion 170 are separably coupled to each other. The power transmitting portion 170 may include a spring 1714 that presses the second rotator 1711 toward the first rotator 1621. The first rotator 1621 and the second rotator 1711 can be separably coupled to each other under the pressing force of the spring 1714. When the input end portion 171 of the power transmitting portion 170 is coupled to a top portion of the first rotator 1621, due to the shapes of the first rotator 1621 and the second rotator 1711, the first rotator 1621 and the second rotator 1711 may not be coupled to each other. If the first rotator 1621 is rotated and thus the first rotator 1621 and the second rotator 1711 are mated with each other so as to mesh with each other, the second rotator 1711 is moved along the rotator guide 1713 in a direction toward the first rotator 1621, and therefore the second rotator can mesh with the first rotator 1621.

The power transmitting portion 170 includes a connecting shaft 1715 inserted to the second rotator 1711, and an input timing pulley 1716 coupled to the connecting shaft 1715. The spring 1714 is disposed between the connecting shaft 1715 and the second rotator 1711, and the connecting shaft 1715 can be partially inserted to the second rotator 1711. The second rotator 1711 and the connecting shaft 1715 may be connected to each other by means of a convex portion and a concave portion. The power transmitting portion 170 includes a drive gear 1721 outputting the rotation power. The drive gear 1721 is coupled to an output timing pulley 1722 rotatably disposed at the output end portion 172. The input timing pulley 1716 and the output timing pulley 1722 are connected to each other by a timing belt 1731 disposed inside the bridge portion 173. The drive gear 1721 can be rotated by the rotation power of the first rotator 1621 through belt power transmission.

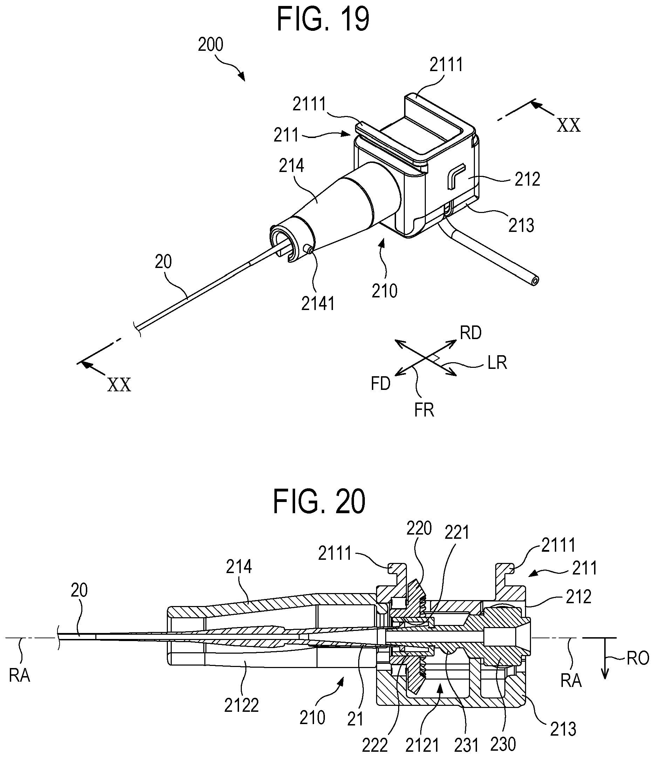

The power transmitting portion 170 has, in the output end portion 172, a pair of fitting grooves 1723 for coupling to the corresponding procedure tool module. The drive gear 1721 is exposed between the pair of fitting grooves 1723. The pair of fitting grooves 1723 are formed in the transverse direction LR. Each of the procedure tool modules has, in its upper end, a pair of fitting protrusions 2111, 3111, 4111, and 5111 *(see* FIGS. 19, 22, 26, and 28), which are fitted to the fitting grooves 1723 in the transverse direction LR. Each of the procedure tool modules can be separably coupled to the power transmitting portion of the corresponding transferring portion through fitting in the transverse direction LR. By way of another example, the orientation of the fitting grooves 1723 and the fitting protrusions 2111, 3111, 4111, and 5111 may be the front and rear direction or an oblique direction between the front and rear direction and the transverse direction. By way of a further example, the power transmitting portion 170 may have the above-described fitting protrusion, and each procedure tool module may have the above-described fitting grooves.

Each of the procedure tool modules includes a driven gear 220, 320, 420, and 520 *(see* FIGS. 19, 22, 26, and 28), which is exposed between the pair of fitting protrusions and meshes with the drive gear 1721 of the power transmitting portion. Each of the procedure tool modules is configured to rotate the procedure tool by the rotation of the driven gear. The drive gear 1721 may be a bevel gear rotating about a rotation axis of the vertical direction, and the driven gear may be a bevel gear rotating about the rotation axis RA of the front and rear direction. By way of another example, a spur gear may be used as the drive gear and the driven gear.

An upper end portion of the rotation power generating portion 160 is fitted to the input end portion 171 of the power transmitting portion 170, and thus can be separably coupled to the input end portion. The rotation power generating portion 160 and the power transmitting portion 170 may be provided with a locking portion. The locking portion may be comprised of an elastically deformable locking latch, and a locking groove to which such a locking latch is separably coupled. One of the locking latch and the locking groove may be provided in the power transmitting portion 170, and the other of the locking latch and the locking groove may be provided in the rotation power generating portion 160.

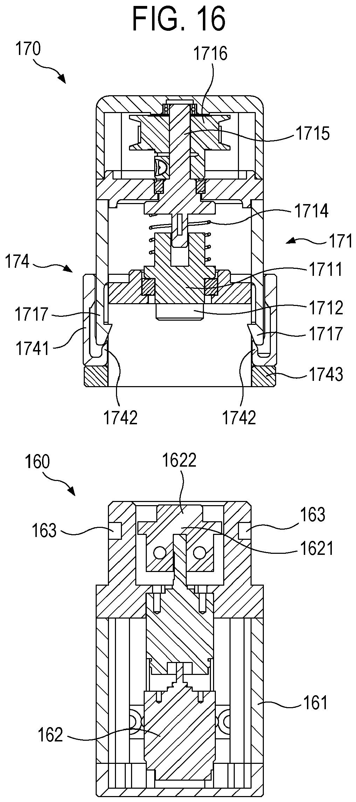

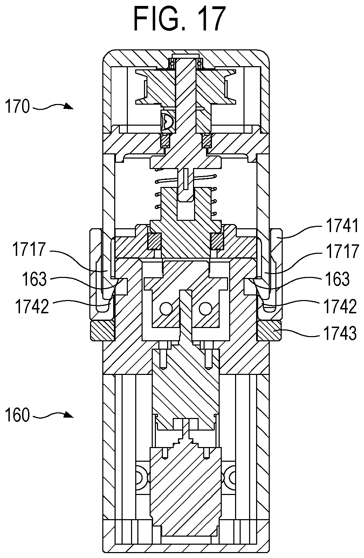

Referring to FIGS. 16 and 17, the power transmitting portion 170 may include a pair of elastically deformable locking latches 1717. The rotation power generating portion 160 includes, in its upper end portion, a locking groove 163 to which the locking latch 1717 is separably coupled. The locking latch 1717 may have a hook shape. If the power transmitting portion 170 is coupled to the rotation power generating portion 160, the locking latch 1717 is separably engaged with the locking groove 163, thereby fixing the power transmitting portion 170 to the rotation power generating portion 160.

Further, a locking releasing portion, which releases the locking latch, may be provided in one of the power transmitting portion provided with the locking latch and the rotation power generating portion. Such a locking releasing portion may be configured to cause the locking latch to be elastically deformed so as to separate the locking latch from the locking groove. Such a locking releasing portion is configured to slide in the vertical direction, and can enhance usage convenience thereby.

Referring to FIGS. 16 and 17, the power transmitting portion 170 including the locking latch 1717 includes a locking releasing portion 174, which is configured to cause the locking latch 1717 to be elastically deformed so as to separate the locking latch 1717 from the locking groove 163 toward the outside of the power transmitting portion. When the power transmitting portion 170 is separated from the rotation power generating portion 160, the locking between the power transmitting portion 170 and the rotation power generating portion 160 can be released by the locking releasing portion 174. The locking releasing portion 174 includes a locking releasing slider 1741 having a locking releasing hook 1742. The locking releasing slider 1741 may be formed to surround an outer peripheral surface of the input end portion 171. The locking releasing slider 1741 may be coupled to the input end portion 171 so as to be slidable in the vertical direction. A stopper 1743 may be fixed to the input end portion 171 below the locking releasing slider 1741. The stopper 1743 may have a shape fitted to the input end portion 171, and prevents the locking releasing slider 1741 from being separated downward from the input end portion 171. When the power transmitting portion 170 and the rotation power generating portion 160 are coupled to each other as shown in FIG. 17, the locking releasing hook 1742 of the locking releasing slider 1741 is positioned between the locking latch 1717 and the locking groove 163. As the locking releasing slider 1741 is slid upward, the locking releasing hook 1742 is inserted between the locking latch 1717 and the locking groove 163 and, at the same time, pushes the locking latch 1717 toward the outside of the input end portion 171 (e.g., in a direction of being spaced apart from the locking groove 163), thereby causing the locking latch 1717 to be elastically deformed. Therefore, the locking latch 1717 is removed from the locking groove 163, and the power transmitting portion 170 can be separated from the rotation power generating portion 160. Accordingly, the locking between the power transmitting portion 170 and the rotation power generating portion 160 can be released.

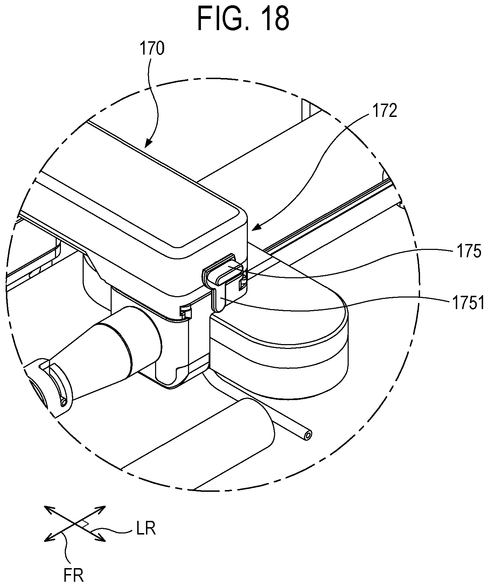

The power transmitting portion 170 may be provided with a latch portion which prevents the separation of the procedure tool module in the transverse direction LR when each of the procedure tool modules is separably coupled to the power transmitting portion 170. Referring to FIG. 18, the power transmitting portion 170 includes a rotating latch 175, which is rotatably disposed in an outer surface of the output end portion 172 and is configured to lock the procedure tool module by rotation. A latching portion 1751 of the rotating latch 175 may be positioned so as to be directed upward or to be directed downward by the rotation of the rotating latch 175. When the power transmitting portion 170 and each procedure tool module are coupled to each other, the rotating latch 175 can be rotated such that the latching portion 1751 locks the procedure tool module.

The vascular intervention procedure device according to one embodiment includes the procedure tool modules coupled to the platform. As described above, the platform selectively transfers the catheter, the guide wire, the micro catheter and the micro guide wire used for the vascular intervention procedure in the front and rear direction, and generates the rotation power for rotating the same about the rotation axis of the front and rear direction. The procedure tool module supports the procedure tool which is one of the catheter, the guide wire, the micro catheter, and the micro guide wire, and can be separably coupled to the platform. Further, the procedure tool module can receive the rotation power from the platform and can rotate the procedure tool.

In one embodiment of the present disclosure, the procedure tool module includes a module housing configured to be separably coupled to the platform. The procedure tool module may include a driven gear, which is disposed in the module housing so as to be rotatable about a rotation axis and rotates the procedure tool. The driven gear is coupled to the procedure tool of each procedure tool module. The driven gear is driven by the rotation power from the platform in the state where the module housing is coupled to the platform, thereby rotating the procedure tool about the rotation axis.

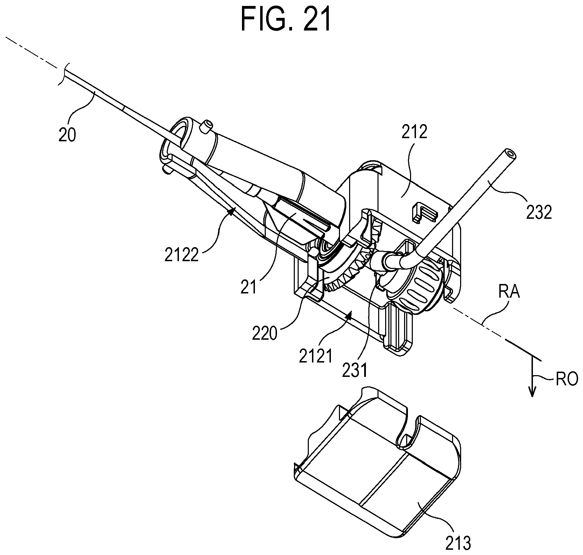

The catheter module is coupled to the transferring portion 130 of the transfer module to be transferred in the front and rear direction, and can rotate the first procedure tool (the catheter). FIG. 19 is a perspective view showing the catheter module of the vascular intervention procedure device according to one embodiment. FIG. 20 is a cross-sectional view taken along line XX-XX of FIG. 19. FIG. 21 is a lower exploded perspective view of the catheter module shown in FIG. 19. Hereinafter, reference is made to FIGS. 4, 5, and 19 to 21.

The catheter module 200 may be attached to and detached from the platform 100 (the transferring portion 130 of the platform) independently while accommodating parts for fixing and rotating the catheter 20. The catheter module 200 includes a first module housing 210, and a first driven gear 220 rotatably disposed in the first module housing 210 and coupled to the first procedure tool.

The first module housing 210 is configured to be separably coupled to the transferring portion 130 (specifically, the power transmitting portion of the transferring portion 130). The first module housing 210 includes a coupling portion 211 coupled to the transferring portion 130 of the platform. The first module housing 210 includes an accommodating portion 212 configured to accommodate the first driven gear 220 and a portion of the catheter 20.

The coupling portion 211 is formed in the transverse direction LR, and is configured to be fitted to the transferring portion 130 of the platform in the transverse direction LR. The coupling portion 211 may include a fitting protrusion 2111, which is formed in the transverse direction LR and is fitted to the fitting groove of the transferring portion 130. The first module housing 210 may be configured such that a portion of the first driven gear 220 is located and exposed in the coupling portion 211.

The accommodating portion 212 may form an internal space of the first module housing 210. The accommodating portion 212 has an opening portion 2121 formed in a radially outward direction RO. The radially outward direction may be a downward direction, but the present disclosure is not limited thereto. Further, the accommodating portion 212 has a front slit 2122, which communicates with the opening portion 2121 and is formed to extend up to a front end of the first module housing 210. The first driven gear 220 and the catheter 20 are configured to be accommodated in the accommodating portion 212 through the opening portion 2121 and the front slit 2122. Further, the first driven gear 220 and the catheter 20 are configured to be separable in the radially outward direction RO through the opening portion 2121 and the front slit 2122.

Further, the first module housing 210 includes a housing cover 213, which is configured to be separably coupled to the accommodating portion 212 so as to open and close the opening portion 2121 of the accommodating portion. For example, the housing cover 213 may be configured to be fitted to the accommodating portion 212 in a radially inward direction opposite to the radially outward direction. The housing cover 213 can open and close a lower end of the accommodating portion 212 opened by the opening portion 2121. In the state where the housing cover 213 is removed from the accommodating portion 212, the first driven gear 220 and the catheter 20 can be separated from the accommodating portion 212 in the radially outward direction RO. The housing cover 213 can be removed from the accommodating portion 212. Thus, when an emergency situation where the operation of the vascular intervention procedure device becomes impossible during the vascular intervention procedure occurs, even in a state where a plurality of procedure tools are maintained in the state of being inserted into the target blood vessel of a patient, the first driven gear 220 and the catheter 20 are separated from the first module housing 210 and the plurality of procedure tools can be manipulated manually.

The first driven gear 220 may be comprised of a single bevel gear. The first driven gear 220 may be disposed in the first module housing 210 so as to mesh with the drive gear 1721 *(see* FIG. 14) of the transferring portion 130. The first driven gear 220 is coupled to the first module housing so as to be rotatable about the rotation axis RA. A central portion of the first driven gear 220 is formed to have a circular through hole 221. A bush 222 is fitted to the through hole 221. A connection end portion 21 of the catheter 20 (the first procedure tool) is fitted to an inner peripheral surface of the bush 222. The first driven gear 220 is coupled, at its central portion, to the connection end portion 21 of the catheter through the bush 222. The catheter 20 can be rotated by the rotation of the first driven gear 220. A portion of the catheter 20 is disposed in the first module housing 210 so as to extend frontward from the central portion of the first driven gear 220.

The catheter module 200 includes a connecting tube 230 connected to the connection end portion 21 of the catheter 20. The connecting tube 230 may be fixed to the housing cover 213. A front end portion of the connecting tube 230 is inserted into the connection end portion 21 of the catheter. The bush 222 is rotatably coupled to the connecting tube. The bush 222 is coupled to a rear portion of the front end portion of the connecting tube 230. When the bush 222 coupled to the first driven gear 220 and the catheter 20 are rotated integrally, the connecting tube 230 is not rotated. The connecting tube 230 has a guide portion 231 and a branch portion 232, which is branched from the guide portion 231 in the radially outward direction in the middle of the guide portion 231. The guide wire or the micro catheter (the second procedure tool) is inserted into the catheter 20 through the guide portion 231 of the connecting tube 230. The branch portion 232 of the connecting tube 230 can be used for injecting a fluidic medical fluid into the target blood vessel or suctioning a blood clot in the target blood vessel in the state where a leading end portion of the catheter 20 reaches the target blood vessel.

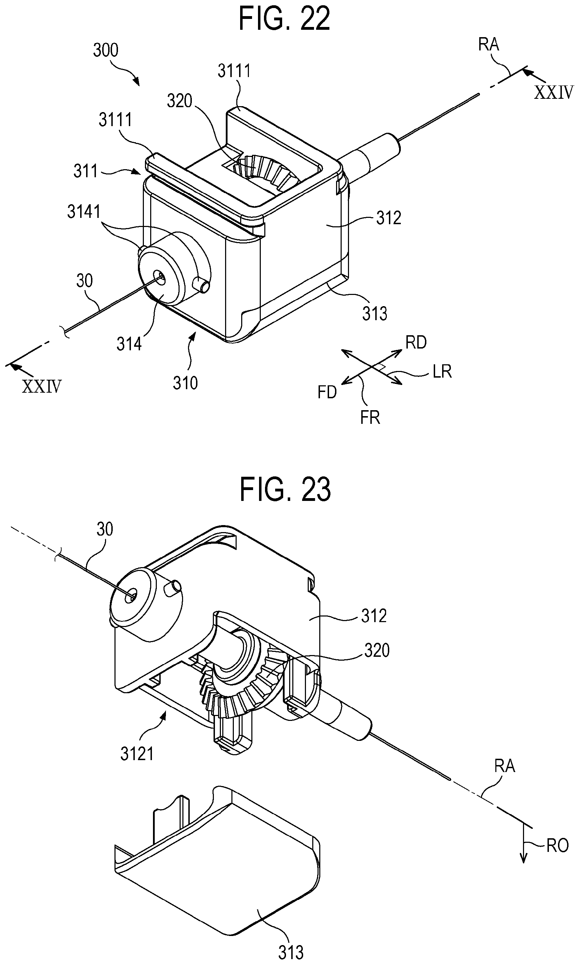

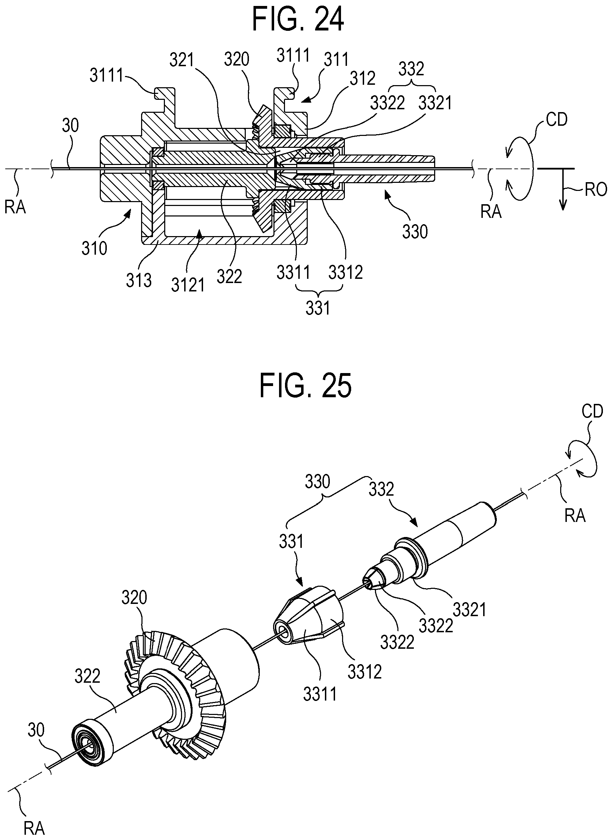

The guide wire module 300 (the second procedure tool module) is coupled to the transferring portion 140 or the transferring portion 150 of the transfer module, and is transferred in the front and rear direction. Further, the guide wire module 300 can rotate the second procedure tool (the guide wire). FIG. 22 is a perspective view showing the guide wire module of the vascular intervention procedure device according to one embodiment. FIG. 23 is a lower exploded perspective view of the guide wire module shown in FIG. 22. FIG. 24 shows a cross-sectional shape taken along line XXIV-XXIV of FIG. 22, and FIG. 25 shows a portion of the guide wire module. Hereinafter, reference is made to FIGS. 4 and 22 to 25.

The guide wire module 300 may be attached to and detached from the platform 100 (the transferring portion 140 of the platform) independently while accommodating parts for fixing and rotating the guide wire 30 (the second procedure tool). The guide wire module 300 includes a second module housing 310, and a second driven gear 320 rotatably disposed in the second module housing 310 and coupled to the guide wire 30.

The second module housing 310 is configured to be separably coupled to the transferring portion 140 or the transferring portion 150. The second module housing 310 includes a coupling portion 311 coupled to the transferring portion 140 or the transferring portion 150 of the platform. The second module housing 310 includes an accommodating portion 312 configured to accommodate the second driven gear 320 and a portion of the guide wire 30.

The coupling portion 311 is formed in the transverse direction LR, and is configured to be fitted to the transferring portion 140 of the platform in the transverse direction LR. The coupling portion 311 may include a fitting protrusion 3111, which is formed in the transverse direction LR and is fitted to the fitting groove of the transferring portion 140 or the fitting groove of the transferring portion 150. The second module housing 310 may be configured such that a portion of the second driven gear 320 is located and exposed in the coupling portion 311.

The accommodating portion 312 may form an internal space of the second module housing 310. The accommodating portion 312 has an opening portion 3121 formed in the radially outward direction RO of the rotation axis RA. The radially outward direction may be a downward direction, but the present disclosure is not limited thereto. The second driven gear 320 is configured to be accommodated in the accommodating portion 312 through the opening portion 3121. The guide wire 30 passes through the second driven gear 320.

Further, the second module housing 310 includes a housing cover 313, which is configured to be separably coupled to the accommodating portion 312 so as to open and close the opening portion 3121 of the accommodating portion. For example, the housing cover 313 is fitted to the accommodating portion 312 in the radially inward direction opposite to the radially outward direction, and can open and close a lower end of the accommodating portion 212 opened by the opening portion 2121. In the state where the housing cover 313 is removed from the accommodating portion 312, the second driven gear 320 can be separated from the accommodating portion 312 in the radially outward direction RO. For example, when the aforementioned emergency situation occurs, even in a state where the plurality of procedure tools are maintained in the state of being inserted into the target blood vessel of a patient and coupled to one another, the second driven gear 320 is separated from the second module housing 310, and the plurality of procedure tools can be manipulated manually.

The second driven gear 320 may be comprised of a single bevel gear. The second driven gear 320 may be disposed in the second module housing 310 so as to mesh with the drive gear 1721 *(see* FIG. 14) of the transferring portion 140 or the transferring portion 150. The second driven gear 320 is coupled to the second module housing so as to be rotatable about the rotation axis RA. A central portion of the second driven gear 320 is formed to have a circular through hole 321, and is coupled to the guide wire 30. The guide wire 30 can be rotated by the rotation of the second driven gear 320. A portion of the guide wire 30 is disposed in the second module housing 310 so as to extend frontward from the central portion of the second driven gear 320.

A guide pipe 322 is coupled to the through hole 321 of the second driven gear 320. The guide pipe 322 is formed such that the guide wire 30 passes through the guide pipe 322. The guide pipe 322 is disposed in the internal space of the accommodating portion 312, and is rotated together with the second driven gear 320.

The guide wire module 300 of one embodiment includes a guide wire holder 330, which is coupled to the second driven gear 320 and is configured to fix the guide wire 30. The guide wire holder 330 is coupled to the through hole 221 formed in the central portion of the second driven gear 320 coaxially with the rotation axis RA of the second driven gear 320. The guide wire holder 330 is configured to releasably fix the guide wire 30.

The guide wire holder 330 includes a clamp holder 331 and a wire clamp 332. The clamp holder 331 is coupled to the through hole 321 of the second driven gear 320 in rear of the guide pipe 322. The guide pipe 322 and the clamp holder 331 may be formed integrally. The wire clamp 332 is configured to releasably fix the guide wire 30, and is coupled to the clamp holder 331 so as to be movable in the front and rear direction FR. The wire clamp 332 is configured to be moved toward the clamp holder 331 to fix the guide wire 30 and to be moved away from the clamp holder 331 to release the guide wire 30.

The clamp holder 331 includes a conical pressing portion 3311 having a diameter increasing in the rearward direction, and a threaded portion 3312 located adjacent to the pressing portion 3311. The wire clamp 332 includes a threaded portion 3321 threadedly coupled to the threaded portion 3312 of the clamp holder, and a plurality of fingers 3322 formed at a front end of the threaded portion 3321 and releasably fixing the guide wire 30. The threaded portion 3321 is coupled to the threaded portion 3312 of the clamp holder so as to be movable through screw motion. As the wire clamp 332 is rotated in one direction, the wire clamp 332 is inserted into the clamp holder 331 frontward. As the wire clamp 332 is rotated in the opposite direction, the wire clamp 332 is moved rearward from the clamp holder 331. The plurality of fingers 3322 are separated in the circumferential direction CD with respect to the rotation axis RA. Further, the plurality of fingers 3322 are elastically deformable in the radially outward direction and the radially inward direction with respect to the rotation axis RA. The plurality of fingers 3322 can make contact with the conical pressing portion 3311.

As the wire clamp 332 is moved frontward, the plurality of fingers 3322 are brought into contact with the conical pressing portion 3311 and are elastically deformed in the radially inward direction, thereby fixing the guide wire 30. As the wire clamp 332 is moved rearward, the plurality of fingers 3322 are elastically deformed in the radially outward direction, thereby releasing the guide wire 30. As the wire clamp 332 is rotated in one direction, the guide wire 30 can be fixed to the second driven gear 320. As the wire clamp 332 is rotated in the opposite direction, the fixing between the guide wire 30 and the second driven gear 320 can be released.

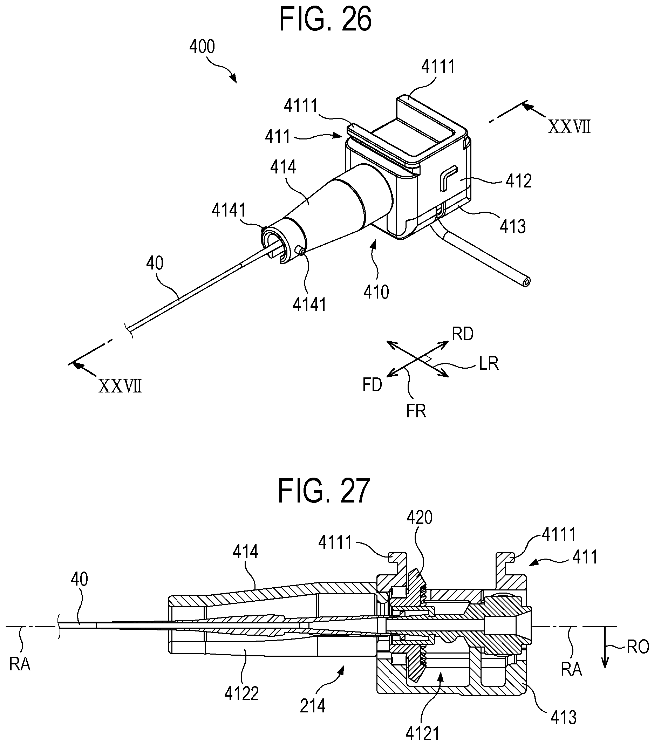

The micro catheter module 400 (the second procedure tool module) is transferred in the front and rear direction by the transferring portion 140 of the transfer module. The micro catheter module 400 is configured to be coupled to the transferring portion 140 of the transfer module so as to be replaceable with the guide wire module 300. Where necessary, the micro catheter module 400 may rotate the micro catheter (the second procedure tool). FIG. 26 is a perspective view showing the micro catheter module of the vascular intervention procedure device according to one embodiment. FIG. 27 is a cross-sectional view taken along line XXVII-XXVII of FIG. 26. Hereinafter, reference is made to FIGS. 5, 26 and 27.

The micro catheter module 400 may be attached to and detached from the platform (the transferring portion 140 of the platform) independently while accommodating parts for supporting the micro catheter 40 (the second procedure tool). In one embodiment, the micro catheter module 400 may have the same structure as the structure of the catheter module 200. Another module, which is configured identically as the catheter module 200, may be configured to support and fix the micro catheter, and may be employed as the micro catheter module 400 in the vascular intervention procedure device.

The micro catheter module 400 includes a third module housing 410, and a third driven gear 420 rotatably disposed in the third module housing 410 and coupled to the micro catheter 40. The third module housing 410 includes a coupling portion 411 fitted to the transferring portion 140 (the fitting groove of the power transmitting portion 170), and an accommodating portion 412 configured to accommodate the third driven gear 420 and a portion of the micro catheter 40. The third module housing 410 and the third driven gear 420 may be configured identically as the first module housing and the first driven gear of the catheter module 200, respectively. An opening portion 4121, a front slit 4122, and a housing cover 413 of the third module housing 410 may be configured identically as the opening portion 2121, the front slit 2122, and the housing cover 213 of the first module housing 210, respectively.

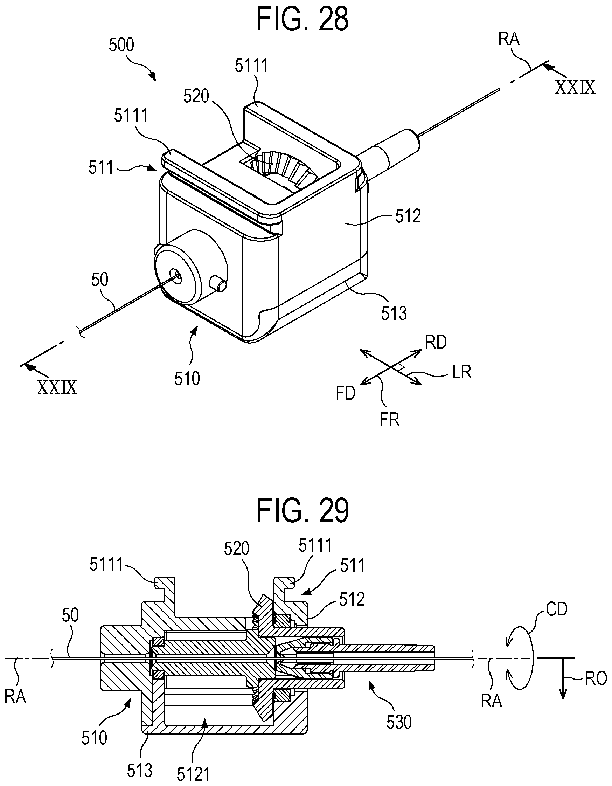

FIG. 28 is a perspective view showing the micro guide wire module of the vascular intervention procedure device according to one embodiment. FIG. 29 is a cross-sectional view taken along line XXIX-XXIX of FIG. 28. Hereinafter, reference is made to FIGS. 5, 28 and 29.

The micro guide wire module 500 may be configured to be coupled to the transferring portion 150 so as to replaceable with the guide wire module 300. Further, the micro guide wire module 500 can rotate the micro guide wire (the third procedure tool) by the rotation power from the transferring portion 150.

The micro guide wire module 500 may be attached to and detached from the platform 100 (the transferring portion 150 of the platform) independently while accommodating parts for fixing and rotating the micro guide wire 50 (the second procedure tool). In one embodiment, the micro guide wire module 500 may have the same structure as the structure of the guide wire module 300. Another module, which is configured identically as the guide wire module 300, may be employed in the vascular intervention procedure device as a module configured to support and rotate the micro guide wire 50.

The micro guide wire module 500 includes a fourth module housing 510, and a fourth driven gear 520 rotatably disposed in the fourth module housing 510 and coupled to the micro guide wire 50. The fourth module housing 510 includes a coupling portion 511 fitted to the transferring portion 150 (the fitting groove of the power transmitting portion 170), and an accommodating portion 512 configured to accommodate the fourth driven gear 520 and a portion of the micro guide wire 50. The fourth module housing 510 and the fourth driven gear 520 may be configured identically as the second module housing and the second driven gear of the guide wire module 300, respectively. An opening portion 5121 and a housing cover 513 of the fourth module housing 510 may be configured identically as the opening portion and the housing cover of the second module housing 310, respectively. Further, the micro guide wire module 500 includes a micro guide wire holder 530, which is coupled to the fourth driven gear 520 and is configured to fix the micro guide wire 50. The micro guide wire holder 530 may be configured identically as the guide wire holder of the guide wire module 300.

The above-described driven gears of each of the modules may comprise a bevel gear. In one embodiment, the module housing of each of the modules is configured to expose a portion of the driven gear, and the driven gear meshes with the drive gear of the transferring portion at its exposed portion. In another embodiment, the module housing of each of the modules may further include a rotation power transmitting portion which is rotatably disposed in the module housing so as to connect with the driven gear. The rotation power transmitting portion may be configured to receive the rotation power generated from the platform in the state of being coupled to the platform (each transferring portion of the platform), and to be connected to the driven gear. By way of example, such a rotation power transmitting portion may have a rotation power transmitting gear meshing with the driven gear at a right angle.

The vascular intervention procedure device according to one embodiment may include a guide module for guiding and supporting the procedure tool being transferred. The guide module is disposed in a guide section, and is configured to guide and support transfer of the procedure tool in the front and rear direction. Since the procedure tool such as the catheter, the guide wire, the micro catheter, and the micro guide wire has flexibility, the procedure tool may sag or bend while being transferred by the transferring force of the transferring portion. The guide module guides and supports the procedure tool so that the procedure tool does not sag or bend during the transfer of the procedure tool, and therefore highly reliable transfer of the procedure tool can be realized.

FIG. 4 shows a first guide section GS 1 located between the front end of the base frame 110 and the catheter module 200, and a second guide section GS2 located between the catheter module 200 and the guide wire module 300 (the second procedure tool module). The vascular intervention procedure device 10 may include a first guide module 600 disposed in the first guide section GS1 and configured to guide and support transfer of the catheter 20 (the first procedure tool) in the front and rear direction, and a second guide module 700 disposed in the second guide section GS2 and configured to guide and support transfer of the guide wire 30 (the second procedure tool) in the front and rear direction.

FIG. 5 shows the first guide section GS 1 located between the front end of the base frame 110 and the catheter module 200, the second guide section GS2 located between the catheter module 200 and the micro catheter module 400 (the second procedure tool module), and a third guide section GS3 located between the micro catheter module 400 and the micro guide wire module 500. The vascular intervention procedure device 10 may include the first guide module 600 disposed in the first guide section GS 1 and configured to guide and support the transfer of the catheter 20 (the first procedure tool) in the front and rear direction, the second guide module 700 disposed in the second guide section GS2 and configured to guide and support transfer of the micro catheter 40 (the second procedure tool) in the front and rear direction, and a third guide module 800 disposed in the third guide section GS3 and configured to guide and support transfer of the micro guide wire 50 (the third procedure tool) in the front and rear direction.

The above-described guide modules 600, 700, and 800 may be attached to and detached from the platform or the procedure tool module individually.

According to one embodiment, each of the guide modules 600, 700, and 800 may include a guide housing disposed at a front end or a rear end of the corresponding guide section, and a pair of supporting members configured to be drawn into or drawn out from the guide housing and configured to sandwich the procedure tool in the transverse direction by being engaged with each other. During the operation of the vascular intervention procedure device 10, lengths of the guide sections GS1, GS2, and GS3 in the front and rear direction are changed. The pair of supporting members are configured to have a variable length in the front and rear direction FR in the guide section according to the length change of the guide section. As the length of each guide section is decreased in the front and rear direction FR, the pair of supporting members can be drawn into the guide housing. As the length of each guide section is increased in the front and rear direction FR, the pair of supporting member can be drawn out from the guide housing.

Each of the guide modules may have a connector at an end portion of the pair of supporting members in a drawn-out direction. The connectors of respective guide modules may be located so as to be opposite to each other in the front and rear direction with respect to the guide housing in the guide section. The guide housing may be disposed at the front end of the each guide section, and the connector may be disposed at the rear end of each guide section. The connector may be disposed at the front end of each guide section, and the guide housing may be disposed at the rear end of each guide section.

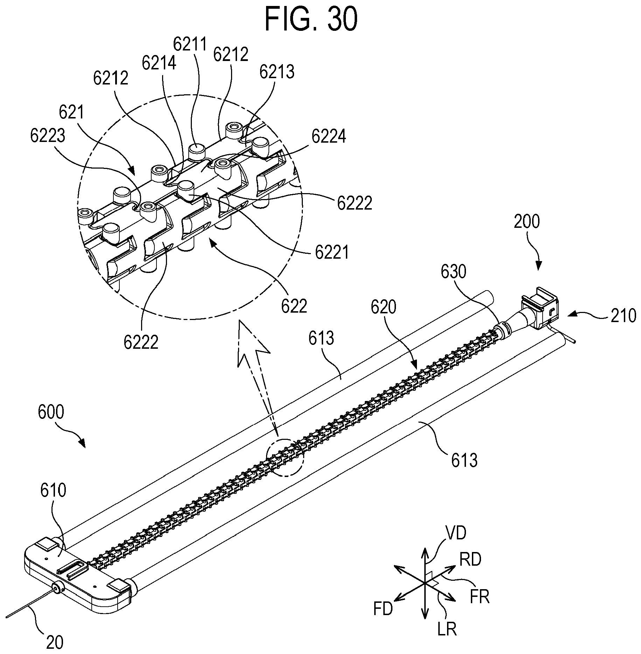

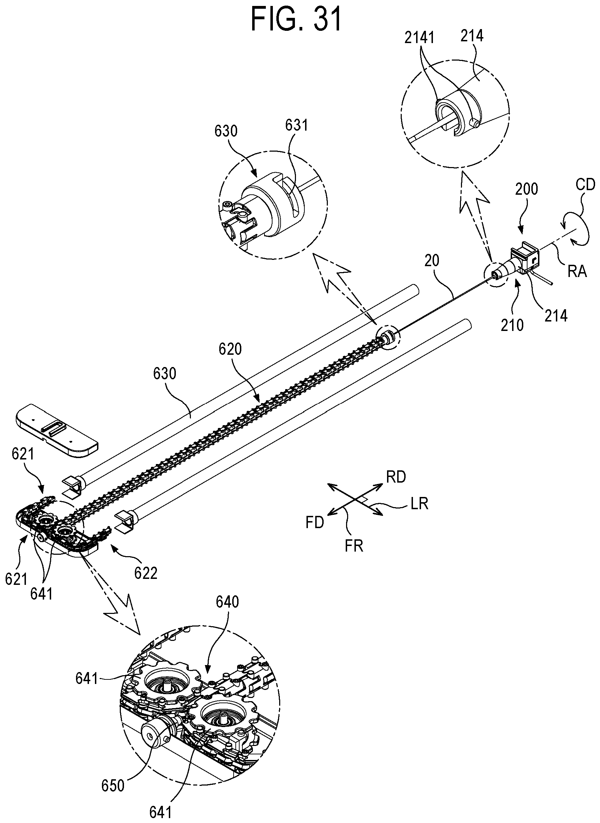

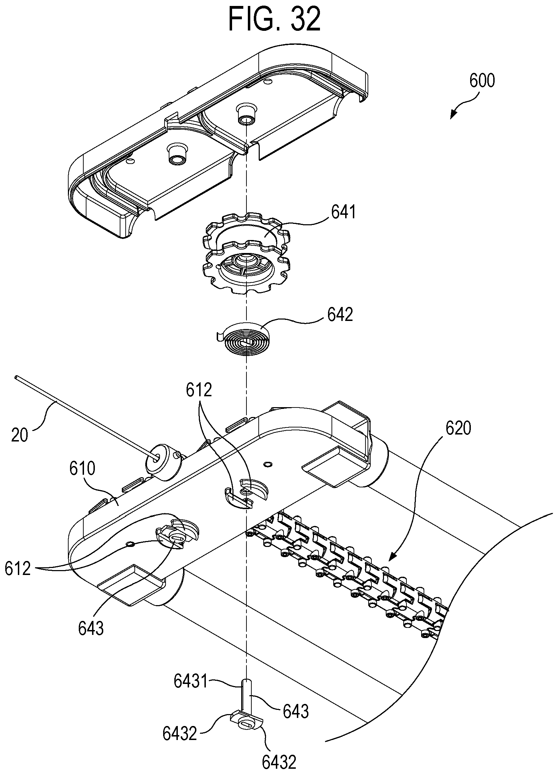

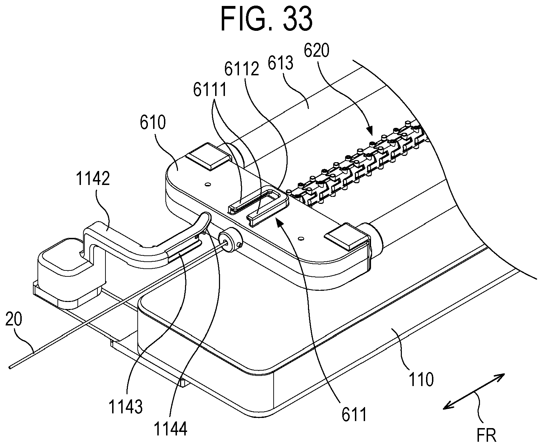

FIG. 30 is a perspective view showing a first guide module of the vascular intervention procedure device according to one embodiment. FIG. 31 is an exploded perspective view of the first guide module shown in FIG. 30. FIG. 32 is a lower exploded perspective view showing a first guide housing of the first guide module shown in FIG. 30. FIG. 33 is a perspective view showing an example where the first guide module shown in FIG. 30 is coupled to the front end of a base frame. Hereinafter, reference is made to FIGS. 4 and 30 to 33.

The vascular intervention procedure device 10 includes the first guide module 600 disposed in the first guide section GS 1, as one of the aforementioned guide modules. The first guide module 600 includes a first guide housing 610 disposed at the front end of the base frame 110 (the front end of the first guide section) and separably mounted to the base frame 110, and a pair of first supporting members 620 configured to be drawn into and drawn out from the first guide housing 610. Further, the first guide module 600 includes a first connector 630, which fixes the end portions of the pair of the first supporting members 620 in a drawn-out direction and is separably coupled to the catheter module 200.

The first guide housing 610 accommodates the pair of first supporting members 620 such that the pair of first supporting members 620 are drawn into and drawn out from the first guide housing. According to one embodiment, the pair of first supporting members 620 comprise a first chain assembly 621 and a second chain assembly 622 which are configured to be engaged with each other in the transverse direction LR. If the first chain assembly 621 and the second chain assembly 622 are engaged with each other in the transverse direction LR, the first and second chain assemblies 621 and 622 sandwich the procedure tool in the state where the procedure tool (e.g., the catheter) is positioned in an internal space between the first chain assembly and the second chain assembly 622.

The first chain assembly 621 is configured such that neighboring links 6212 are connected by a pin 6211. The first chain assembly 621 has engagement teeth 6213 and engagement grooves 6214 which are disposed alternately. The engagement teeth 6213 may be formed in the respective links 6212, and the engagement grooves 6214 may be formed between the neighboring links 6212. The second chain assembly 622 is configured such that neighboring links 6222 are connected by a pin 6221. The second chain assembly 622 has engagement grooves 6223 corresponding to the respective engagement teeth 6213 of the first chain assembly, and engagement teeth 6224 corresponding to the engagement grooves 6214 of the first chain assembly. The engagement grooves 6223 and the engagement teeth 6224 are disposed alternately. The pins 6211 and 6221 of the first and second chain assemblies 621 and 622 are positioned in the vertical direction. The neighboring links of each chain assembly can be rotated in the transverse direction LR about an axis of the pin 6211 and 6221 relatively to each other.

The first guide module 600 includes a first engaging portion 640, which is disposed in the first guide housing 610 and is configured to cause the pair of first supporting members to be engaged with each other. The first engaging portion 640 is configured to make contact with each of the pair of first supporting members 620 and to cause the pair of first supporting members 620 to be engaged with each other in the transverse direction LR when the pair of first supporting members 620 are drawn out from the first guide housing 610 and the variable length of the first guide section GS 1 is increased. Further, the first engaging portion 640 may disengage the pair of first supporting members 620 from each other in the transverse direction LR when the pair of first supporting members 620 are drawn into the first guide housing 610.

The first engaging portion 640 includes a pair of first engaging wheels 641 which are in contact with the pair of first supporting members 620 respectively and are rotated thereby. The pair of first engaging wheels 641 are spaced apart from each other by a distance that permits the pair of the first supporting members 620 to be engaged with each other between the pair of first engagement wheels. The pair of first engaging wheels 641 are sprocket wheels having grooves that are engaged with the pins 6211 and 6221 of the first and second chain assemblies, respectively. The pair of first engaging wheels 641 are rotated by the pair of first supporting members 620 when the pair of first supporting members 620 are drawn into the first guide housing 610 and are drawn out from the first guide housing 610.

As the length of the guide section in the front and rear direction is changed according to the transfer of the procedure tool in the front and rear direction, the pair of first supporting members 620 can be drawn into and drawn out from the first guide housing 610. As the pair of first supporting members 620 are drawn out from the first guide housing 610, the pair of first supporting members 620 can be engaged with each other by the pair of first engaging wheels 641. As the pair of first supporting members 620 are drawn into the first guide housing 610, the pair of first supporting members 620 passing through the pair of first engaging wheels 641 are disengaged from each other.

The first engaging portion 640 includes springs 642 which press the pair of first engaging wheels 641, respectively, in a direction where the pair of first supporting members 620 are drawn into the first guide housing 610. The spring 642 may be a spiral spring. An inward end of the spring 642 is coupled to a slit 6431 formed in a spring shaft 643 in the vertical direction. An outward end of the spring 642 is coupled to an inner peripheral surface of the first engaging wheel 641. The spring 642 is disposed between the first engaging wheel 641 and the spring shaft 643 in the state being wound in advance in a drawn-in direction of the pair of the first supporting members 620. The first engaging wheels 641 can be pressed in the drawn-in direction of the pair of first supporting members 620. Since the first engaging wheels 641 are pressed, in the free state of the first guide module 600, the pair of first supporting members 620 may have a minimal protruding length from the first guide housing 610. Further, when the pair of first supporting members 620 are drawn into the first guide housing 610, the pressing force of the springs 642 can assist the drawn-in operation of the pair of first supporting members 620.

The restoring force of the pair of springs 642 in the first guide housing 610 can be adjusted. The spring shaft 643 coupled to each of the pair of springs 642 may be configured to adjust the restoring force of the spring 642. The spring shaft 643 may have the slit 6431 formed in an elongated manner in the vertical direction. The spring shaft 643 may be separated downward in the state of accommodating one end of the spring 642. The spring shaft 643 has, at its lower end, a pair of protruding pieces 6432. Further, a pair of semi-circular protruding portions 612, to which the pair of protruding pieces 6432 are inserted respectively, protrude downward in a lower surface of a bottom portion of the first guide housing 610. If the pair of protruding pieces 6432 are positioned between the pair of protruding portions 612 by the rotation of the spring shaft 643, the spring shaft 643 can be extracted downward from the first guide housing 610. In the state where the spring shaft 643 is extracted from the first guide housing 610, the restoring force of the spring 642 can be adjusted by the rotation of the spring shaft 643, and the pressing force of the spring 642 can be adjusted thereby. After the restoring force of the spring 642 is adjusted to a desired extent, the spring shaft 643 is inserted into the first guide housing 610, and the pair of protruding pieces 6432 are fitted to the pair of protruding portions 612, respectively. The spring 642 can be disposed in the first guide housing 610 in the state of having the adjusted restoring force.

Further, the first guide housing 610 includes a pair of tubes 613 extending rearward from the first guide housing 610. The portions of the pair of first supporting members 620, which are disengaged from each other while passing through the first engaging wheels 641, can be inserted into the pair of tubes 613, respectively.

The first guide module 600 may further include a splitting portion 650 which is configured to split the pair of first supporting members 620 when the pair of first supporting members 620 are drawn into the first guide housing 610. The splitting portion 650 may be disposed between the pair of first supporting members 620 in the first guide housing 610. The splitting portion 650 may be configured to allow the catheter to pass through the splitting portion in the front and rear direction FR.

The first connector 630 of the first guide module 600 may be separably coupled to the first module housing 210 of the catheter module 200. Referring to FIG. 31, the first connector 630 is formed as a female part. The first connector 630 may be comprised of two halves which can sandwich the end portions of the pair of first supporting members 620 in the transverse direction LR. The first connector 630 has a pair of fitting slits 631 formed in the circumferential direction CD. The first connector 630 is separably coupled to the connecting portion 214 formed at the front end of the first module housing 210. The connecting portion 214 is a male part. The front slit 2122 of the first module housing *(see* FIG. 21) is formed in the connecting portion 214. The connecting portion 214 has a fitting pin 2141 fitted to the fitting slit 631, and is fitted to the first connector 630. Due to the fitting slit 631 and the fitting pin 2141, the first connector 630 and the pair of first supporting members 620 can be positioned at a correct position with respect to the catheter module 200, and can be easily coupled to the catheter module 200.

The first guide housing 610 is separably coupled to the front end of the base frame 110. Referring to FIG. 33, the housing holder 1142 of the base frame 110 has the fitting groove 1143 formed in the front and rear direction FR, and a hook 1144 which is formed at a rear end of the fitting groove 1143 and is elastically deformable. The first guide housing 610 has, on its top portion, a U-shaped holder coupling portion 611 to which the housing holder 1142 is fitted rearward. A pair of protrusions 6111 of the holder coupling portion 611 are fitted to the fitting groove 1143. The hook 1144 is engaged with an engagement portion 6112 of the holder coupling portion 611 between the pair of protrusions 6111, thereby locking the first guide housing 610 to the housing holder 1142. When the first guide housing 610 is removed from the housing holder 1142, the hook 1144 is pushed upward, and the hook 1144 can be disengaged from the engagement portion 6112.

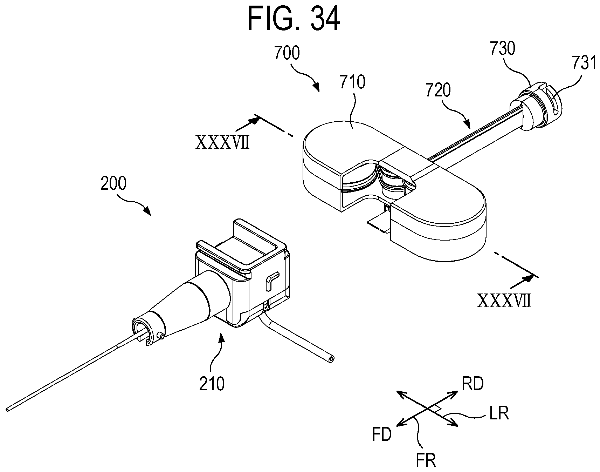

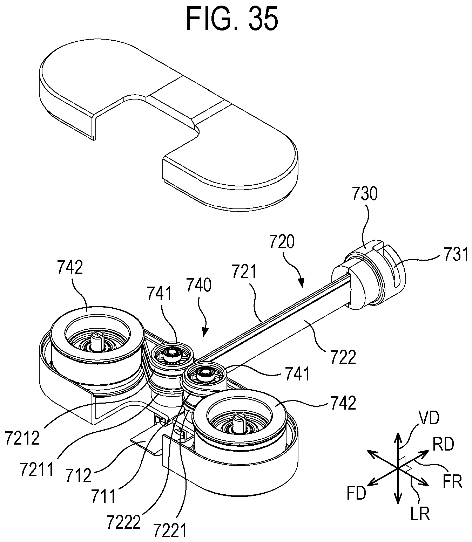

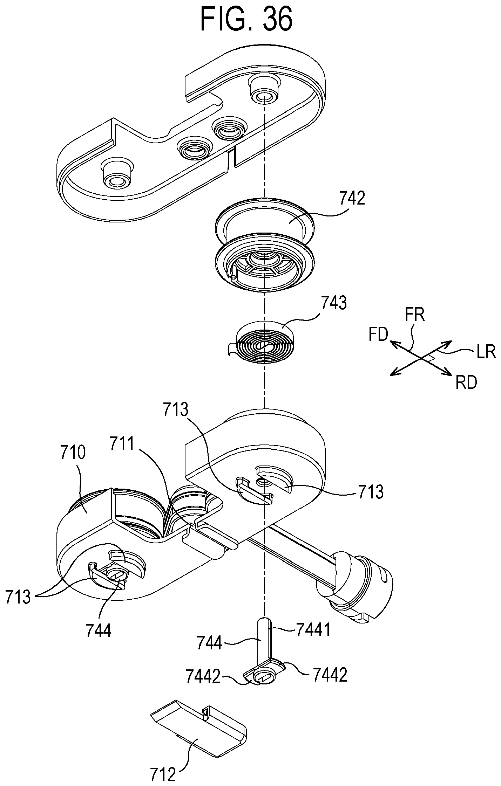

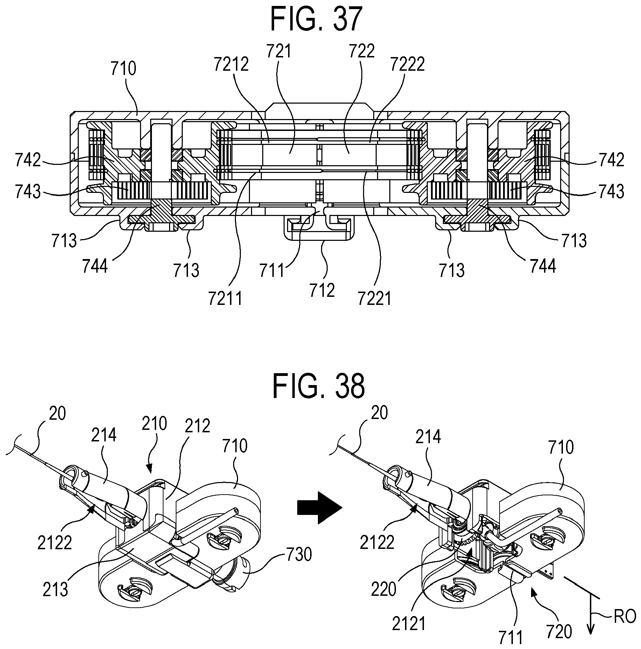

FIG. 34 is a perspective view showing the second guide module of the vascular intervention procedure device according to one embodiment. FIG. 35 is an exploded perspective view of the second guide module shown in FIG. 34. FIG. 36 is a lower exploded perspective view of the second guide module shown in FIG. 34. FIG. 37 is a cross-sectional view taken along line XXXVII-XXXVII of FIG. 34. Hereinafter, reference is made to FIGS. 4, 5, and 34 to 37.

The vascular intervention procedure device 10 includes the second guide module 700 disposed in the second guide section GS2, as one of the aforementioned guide modules. The second guide module 700 includes a second guide housing 710 disposed at the front end of the second guide section GS2 and separably mounted to the catheter module 200, and a pair of second supporting members 720 configured to be drawn into and drawn out from the second guide housing 710. Further, the second guide module 700 includes a second connector 730, which fixes the end portions of the pair of the second supporting members 720 in the drawn-out direction and is separably coupled to the guide wire module 300 or the micro catheter module 400.

The second guide housing 710 accommodates the pair of second supporting members 720 such that the pair of second supporting members 720 are drawn into and drawn out from the second guide housing. According to one embodiment, the pair of second supporting members 720 comprise a first band 721 and a second band 722, which are configured to be engaged with each other in the transverse direction LR. If the first band 721 and the second band 722 are engaged with each other in the transverse direction LR, the first and second bands 721 and 722 sandwich the procedure tool in the state where the procedure tool (e.g., the guide wire or the micro catheter) is positioned in an internal space between the first and second bands 721 and 722.

The first band 721 has, in its surface facing the second band 722, an engagement protrusion 7211 formed in the front and rear direction FR. The engagement protrusion 7211 may extend continuously in the front and rear direction FR. Alternatively, the engagement protrusion 7211 may be formed as a plurality of engagement protrusions disposed intermittently. The second band 722 has, in its surface facing the first band 721, an engagement groove 7221 to which the engagement protrusion 7211 is fitted. The first band 721 may have the engagement protrusion 7211 and an engagement groove 7212 together, and the second band 722 may have the engagement groove 7221 corresponding to the engagement protrusion 7211 of the first band, and an engagement protrusion 7222 corresponding to the engagement groove 7212 of the first band. The first and second bands 721 and 722 are disposed such that their width direction lie on the vertical direction VD.

The second guide housing 710 is separably coupled to the catheter module 200. The second guide housing 710 is separably coupled to the first module housing 210 of the catheter module 200. The second guide housing 710 has, at its lower side, a rear slit 711 formed in the front and rear direction FR. The rear slit 711 is formed to communicate with the opening portion 2121 of the first module housing *(see* FIG. 21) and to extend from the opening portion 2121 to the rear end. By way of another example, the first module housing 210 of the catheter module may be configured to have the aforementioned rear slit 711. Alternatively, in the state where the first module housing 210 of the catheter module and the second guide housing 710 of the second guide module are coupled integrally, the first module housing 210 may be configured to have the aforementioned rear slit 711.

The second guide housing 710 includes a cover latch 712 which is slidably fitted to the second guide housing 710 through the rear slit 711. In the state where the second guide housing 710 is coupled to the first module housing 210, the cover latch 712 supports the housing cover 213 *(see* FIG. 21), and therefore can fix the housing cover 213 to the accommodating portion 212. The cover latch 712 can be separated from the second guide housing 710, and therefore the housing cover 313 can be separated from the accommodating portion 312.

The second guide module 700 includes a second engaging portion 740, which is disposed in the second guide housing 710 and is configured to cause the pair of second supporting members to be engaged with each other. The second engaging portion 740 is configured to make contact with each of the pair of second supporting members 720 and to cause the pair of second supporting members 720 to be engaged with each other in the transverse direction LR when the pair of second supporting members 720 are drawn out from the second guide housing 710 while the variable length of the second guide section GS2 is increased. Further, the second engaging portion 740 may disengage the pair of second supporting members 720 from each other in the transverse direction LR when the pair of second supporting members 720 are drawn into the second guide housing 710.

The second engaging portion 740 includes a pair of second engaging wheels 741, which are in contact with the pair of second supporting members 720, respectively, and are rotated thereby. The pair of second engaging wheels 741 are rollers that make contact with the first and second bands 721 and 722, respectively, and can be rotated thereby. The pair of second engaging wheels 741 are rotated by the pair of second supporting members 720 when the pair of second supporting members 720 are drawn into the second guide housing 710 and are drawn out from the second guide housing 710. Further, the second engaging portion 740 includes a pair of reels 742, and the second supporting members passing through the second engaging wheels 741 are wound around and unwound from the pair of reels, respectively. The pair of reels 742 are disposed at the outside of the second engaging wheels 741, respectively.

As the length of the guide section in the front and rear direction is changed according to the transfer of the procedure tool in the front and rear direction, the pair of second supporting members 720 can be drawn into and drawn out from the second guide housing 710. As the pair of second supporting members 720 are drawn out from the second guide housing 710, the pair of second supporting members 720 can be engaged with each other by the pair of second engaging wheels 741. As the pair of second supporting members 720 are drawn into the second guide housing 710, the pair of second supporting members 720 passing through the pair of second engaging wheels 741 are disengaged from each other.

The second engaging portion 740 includes springs 743 which press the pair of second engaging wheels 741, respectively, in a direction where the pair of second supporting members 720 are drawn into the second guide housing 710. The springs 743 may be spiral spring. An inward end of the spring 743 is coupled to a slit 7441 formed in a spring shaft 744 in the vertical direction. An outward end of the spring 743 is coupled to an inner peripheral surface of the reel 742. A pair of the springs 743 are disposed between the respective reels 742 and the respective spring shafts 744 in the state being wound in advance in a drawn-in direction of the pair of the second supporting members 720, and the pair of reels 742 are connected to the second engaging wheels 741 through the first and second bands 721 and 722, respectively. The second engaging wheel 741 can be pressed in the drawn-in direction of the pair of second supporting members 720 by the spring 743. Since the second engaging wheel 741 is pressed, in the free state of the second guide module 700, the pair of second supporting members 720 may have a minimal protruding length from the second guide housing 710. Further, when the pair of second supporting members 720 are drawn into the second guide housing 710, the pressing force of the spring 743 can assist the drawn-in operation of the pair of second supporting members 720.

The restoring force of the pair of springs 743 in the second guide housing 710 can be adjusted. The spring shaft 744 coupled to each of the pair of springs 743 may be configured to adjust the restoring force of the spring 743. After the restoring force of the spring 743 is adjusted to a desired extent, the spring shaft 744 is inserted into the second guide housing 710, and a pair of protruding pieces 7442 are fitted to a pair of protruding portions 713, respectively. The method of adjusting the restoring force of the spring 743 by means of the spring shaft 744 may be identical to the method of adjusting the restoring force by the spring shaft 643 in the first guide module.

The second connector 730 of the second guide module 700 may be coupled to the second module housing of the guide wire module 300 or the third module housing of the micro catheter module. Referring to FIG. 34, the second connector 730 is formed as a female part. The second connector 730 may be comprised of two halves which can sandwich the end portions of the pair of second supporting members 720 in the transverse direction LR. The second connector 730 has a pair of fitting slits 731 formed in the circumferential direction CD. The second connector 730 is separably coupled to the connecting portion 314 formed at the front end of the second module housing *(see* FIG. 22) or the connecting portion 414 formed at the front end of the third module housing *(see* FIG. 26). The connecting portions 314 and 414 are a male part. The front slit 4122 of the third module housing is formed in the connecting portion 414. The connecting portions of the second and third module housings have fitting pins 3141 and 4141 fitted to the fitting slit 731, and are fitted to the second connector 730. Due to the fitting slit 731 and the fitting pins 3141 and 4141, the second connector 730 and the pair of second supporting members 720 can be positioned at a correct position with respect to the guide wire module or the micro catheter module, and can be easily coupled to the guide wire module or the micro catheter module.

Referring to FIG. 38, the front slit 2122, which communicates with the opening portion 3121 of the first module housing 210, is formed in the connecting portion 214 of the first module housing 210. The front slit 2122 is formed in a lower side of the connecting portion 214, and is formed to permit the passage of a portion of the catheter 20 located in the connecting portion 214. In the state where the cover latch of the second guide housing 710 is removed, the housing cover 213 of the first module housing 210 can be removed, and the rear slit 711 of the second guide housing 710 can be exposed. Further, the second connector 730 can be separated from the pair of second supporting members 720. The reels in the second guide housing 710 are pressed in the drawn-in direction of the pair of second supporting members 720. If the second connector 730 is separated from the end portions of the pair of second supporting members 720, the pair of second supporting members 720 can be completely wound around the reels, respectively, and the guide wire can be exposed. The housing cover 213 may be configured to be separated from the first module housing 210, and the second connector 730 may be configured to be separated from the pair of second supporting members 720. The catheter 20 and the first driven gear 220 can be separated from the accommodating portion 212 through the front slit 2122 and the opening portion 2121, and the guide wire inserted into the catheter 20 can be separated from the accommodating portion 212 through the rear slit 711 of the second guide housing 710 in the radially outward direction RO.

Referring to FIGS. 5 and 30 to 37, the vascular intervention procedure device 10 may include the first, second, and third guide modules 600, 700, and 800 that are disposed in the first, second, and third guide sections GS1, GS2, and GS3, respectively. In the second operation mode of the vascular intervention procedure device 10, the first, second, and third guide modules 600, 700, and 800 can be employed. In the second operation mode of the vascular intervention procedure device 10 shown in FIG. 5, the second guide module 700 guides and supports the transfer of the micro catheter 40 of the micro catheter module 400. The third guide module 800 guides and supports the transfer of the micro guide wire 50 and the micro guide wire module 500.

The second guide module 700 in the first operation mode shown in FIG. 4 is used as the second guide module in the second operation mode shown in FIG. 5. The second guide housing 710 of the second guide module 700 is disposed at the front end of the second guide section GS2, and is separably coupled to the catheter module 200. The second connector 730 of the second guide module 700 is disposed at the rear end of the second guide section GS2, and is separably coupled to the micro catheter module 400. The transfer of the micro catheter at the second guide section GS2 is guided and supported by the second guide module 700.

The third guide module 800 has the same configuration as the configuration of the second guide module 700. That is, a third guide housing 810, a pair of third supporting members 820, and a third connector 830 of the third guide module 800 may have the same configuration as the second guide housing 710, the pair of second supporting members 720, and the second connector 730 of the second guide module 700, respectively. Further, a third engaging portion of the third guide module 800 has the same configuration as the configuration of the second engaging portion 740 of the second guide module 700. A pair of third supporting members 820 comprised of the above-described first and second bands guide and support the transfer of the procedure tool (e.g., the micro guide wire) at the third guide section GS3.

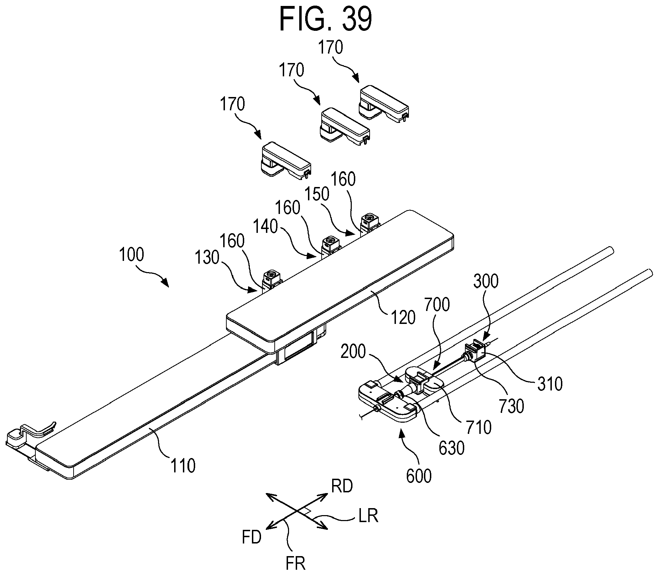

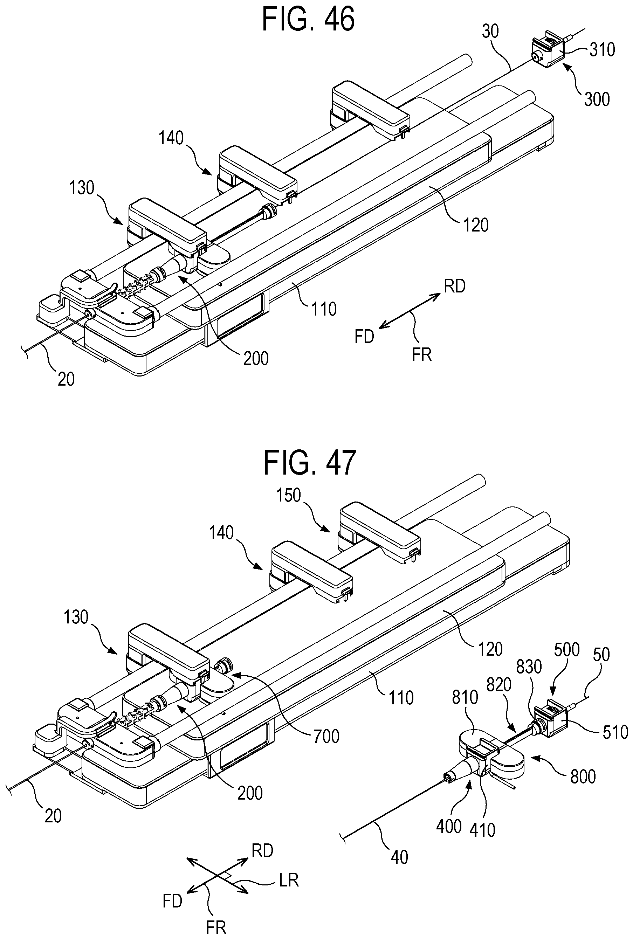

Operation examples of the vascular intervention procedure device according to one embodiment are described with reference to FIGS. 39 to 52. FIGS. 39 to 46 show an operation example of the vascular intervention procedure device of one embodiment in the first operation mode. FIGS. 47 to 52 show an operation example of the vascular intervention procedure device of one embodiment in the second operation mode.

Referring to FIG. 39, the platform 100 is in a ready state before operation, and the catheter module 200 (the first procedure tool module in a first operation state) and the guide wire module 300 (the second procedure tool module in the first operation state) are prepared. The first connector 630 of the first guide module 600 is coupled to the catheter module 200, and the pair of first supporting members of the first guide module 600 may be drawn into the first guide housing of the first guide module 600. The second guide housing 710 of the second guide module 700 is coupled to the catheter module 200. The second connector 730 of the second guide module 700 is coupled to the second module housing 310 of the guide wire module 300, and the guide wire module 300 is connected to the catheter module 200 through the second guide module 700. Further, each of the power transmitting portions 170 is fitted to the corresponding rotation power generating portion 160.

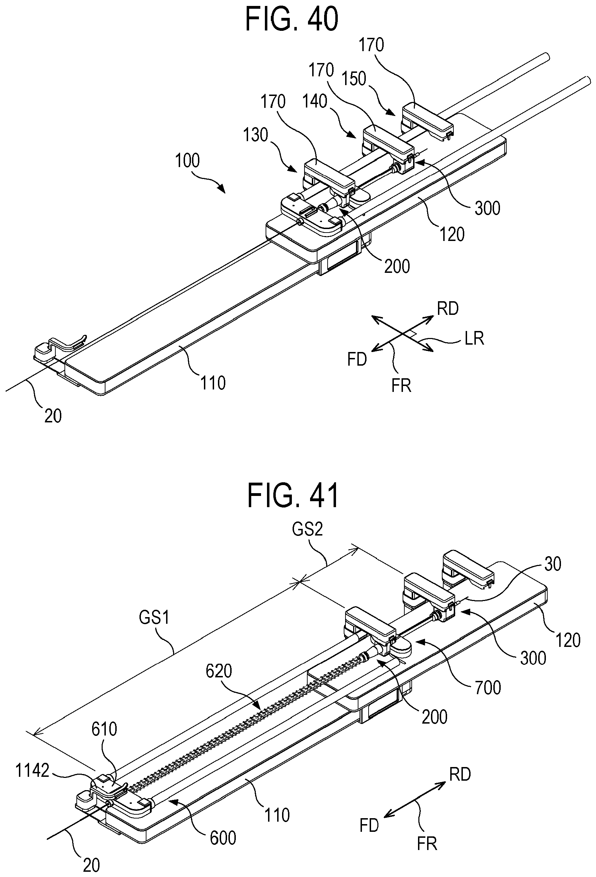

Referring to FIG. 40, the catheter module 200 is fitted to the power transmitting portion 170 of the transferring portion 130 in the transverse direction LR, and the guide wire module 300 is fitted to the power transmitting portion 170 of the transferring portion 140 in the transverse direction LR. The guide wire module 300 may be fitted to the power transmitting portion 170 of the transferring portion 150. In such a case, the procedure tool module is not coupled to the transferring portion 140.

Referring to FIG. 41, the first guide housing 610 of the first guide module 600 is coupled to the housing holder 1142 disposed at the front end of the base frame 110. The first guide housing 610 is pulled frontward from the catheter module 200. Further, the pair of first supporting members 620 are drawn out from the first guide housing 610, and sandwich the catheter 20 in the state of being engaged with each other at the first guide section GS 1. The second guide module 700 is disposed at the second guide section GS2 and sandwiches the guide wire 30.

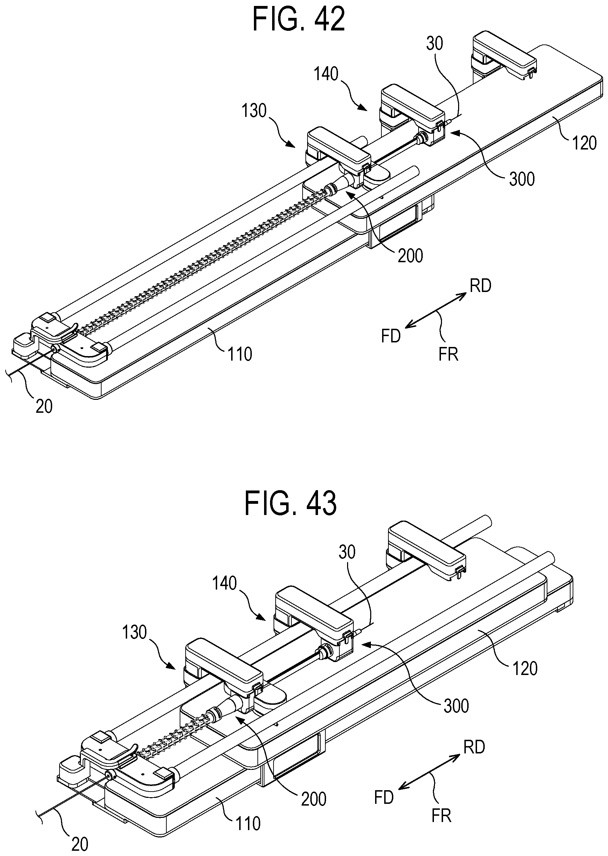

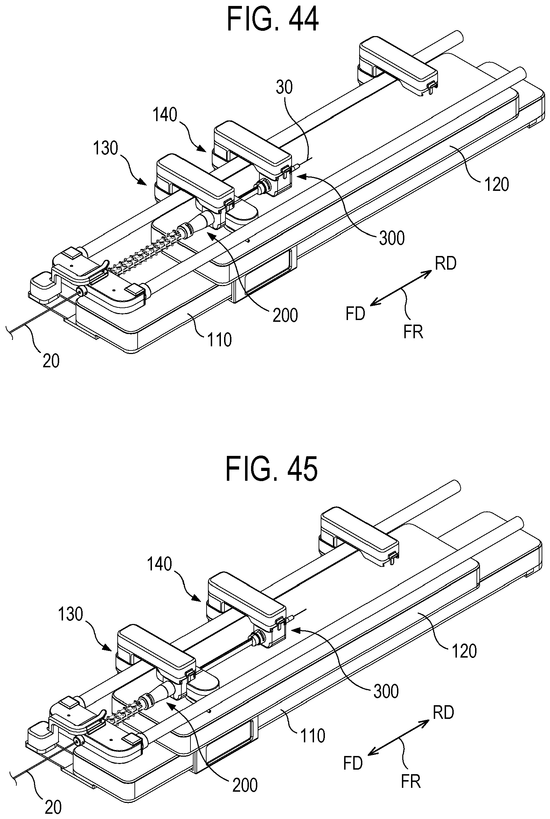

Referring to FIG. 42, the transferring portions 130 and 140 are transferred, and thereby the catheter module 200 and the guide wire module 300 are simultaneously transferred frontward. Therefore, the catheter 20 and the guide wire 30 can be transferred frontward. Referring to FIG. 43, the transfer frame 120 of the transfer module is transferred frontward. Therefore, the catheter 20 and the guide wire 30 can be transferred frontward. Referring to FIG. 44, the transferring portion 140 is further transferred frontward, and thereby the guide wire 30 can be further transferred frontward. Referring to FIG. 45, the transfer frame 120 is further transferred frontward, the transferring portion 140 is transferred rearward, and therefore the catheter 20 can be introduced into the target blood vessel along the guide wire 30. In the examples shown in FIGS. 42 to 45, the catheter module 200 can rotate the catheter 20 by the rotation power from the transferring portion 130, and the guide wire module 300 can rotate the guide wire 30 by the rotation power from the transferring portion 140. Referring to FIG. 46, in the state where the catheter 20 reaches the target blood vessel, the guide wire 30 is removed from the catheter 20. The second module housing 310 of the guide wire module 300 is separated from the transferring portion 140, and the guide wire module 300 is moved rearward. Therefore, the guide wire 30 is removed from the catheter 20.

FIG. 47 shows an initial state in the second operation mode of the vascular intervention procedure device of one embodiment. The catheter 20 reaches the target blood vessel, and the catheter module 200 (the first procedure tool module in the second operation state) and the transfer frame 120 are stationary. To couple the micro catheter module 400 to the transferring portion 140, the transferring portion 140 is moved rearward. The micro catheter module 400 (the second procedure tool module in the second operation state) and the micro guide wire module 500 (the third procedure tool module in the second operation state) are prepared. The micro catheter 40 extends frontward from the micro catheter module 400. The third guide housing 810 of the third guide module 800 is coupled to the third module housing 410 of the micro catheter module 400. The third connector 830 of the third guide module 800 is coupled to the fourth module housing 510 of the micro guide wire module 500, and the micro guide wire module 500 is connected to the micro catheter module 400 through the third guide module 800. The micro guide wire 50 is inserted into the micro catheter 40 through the micro catheter module 400 in the state of being supported by the pair of third supporting members 820.

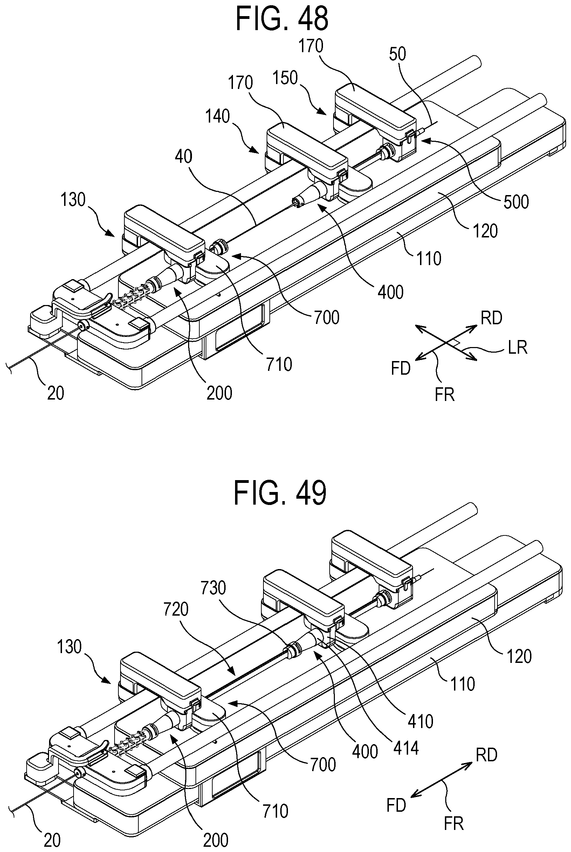

Referring to FIG. 48, the micro catheter module 400 is fitted to the power transmitting portion 170 of the transferring portion 140 in the transverse direction LR, and the micro guide wire module 500 is fitted to the power transmitting portion 170 of the transferring portion 150 in the transverse direction LR. The pair of second supporting members of the second guide module 700 are drawn into the second guide housing 710. The micro catheter 40 and the micro guide wire 50 are inserted between the pair of second supporting members of the second guide module 700 through the second connector 730 of the second guide module 700, and, thereafter, are inserted to the catheter 20. Referring to FIG. 49, the pair of second supporting members 720 of the second guide module 700 are drawn out from the second guide housing 710 rearward, and the second connector 730 of the second guide module 700 is coupled to the connecting portion 414 formed at the front end of the third module housing 410 of the micro catheter module 400.

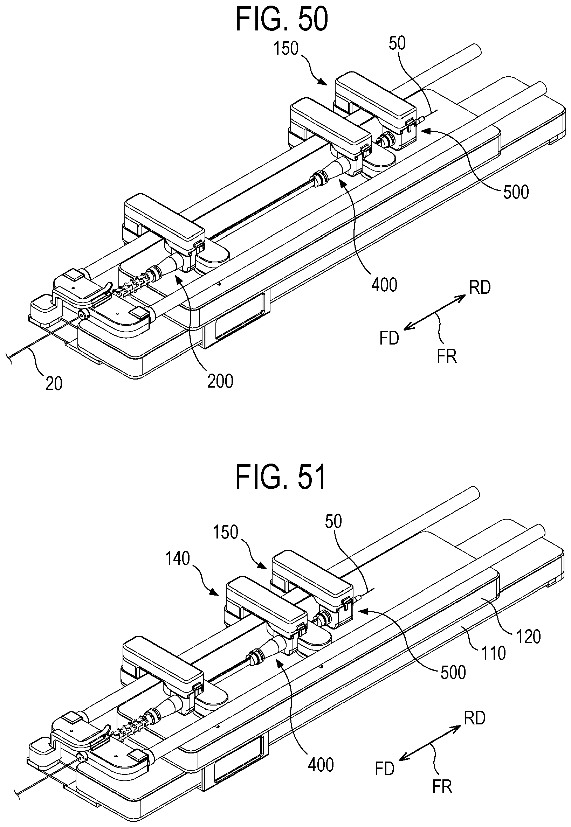

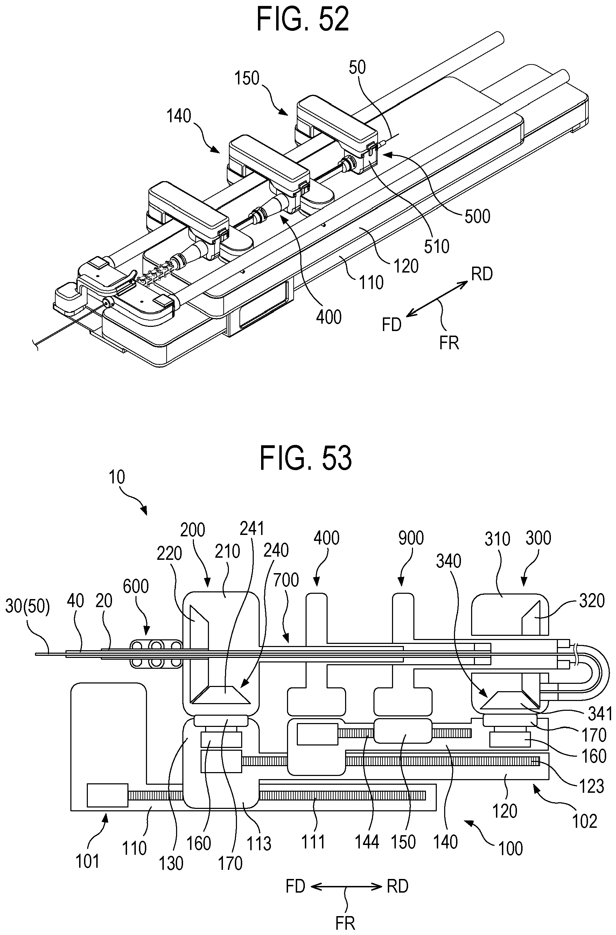

Referring to FIG. 50, the transferring portion 150 is transferred frontward, and thereby the micro guide wire 50 is transferred frontward in the state of being inserted into the micro catheter 40. Referring to FIG. 51, the transferring portions 140 and 150 are simultaneously transferred frontward, and thereby the micro catheter and the micro guide wire 50 are simultaneously transferred frontward. In the examples shown in FIGS. 50 and 51, the micro guide wire module 500 can rotate the micro guide wire 50 by the rotation power from the transferring portion 150. Further, where necessary, the micro catheter module 400 may rotate the micro catheter by the rotation power from the transferring portion 140. Referring to FIG. 52, in the state where the micro guide wire 50 reaches the target blood vessel, the transferring portion 140 is further transferred frontward. Therefore, the micro catheter can be introduced into the target blood vessel along the micro guide wire 50. If the micro catheter is introduced into the target blood vessel, the fourth module housing 510 of the micro guide wire module 500 is removed from the transferring portion 150, and the micro guide wire module 500 is transferred rearward. Therefore, the micro guide wire 50 is removed from the micro catheter.

As shown in FIGS. 42 to 52, the vascular intervention procedure device 10 according to one embodiment performs an operation having at least five degrees of freedom. The aforesaid five degrees of freedom include three degrees of freedom related to the insertion and movement of the procedure tool, and two degrees of freedom related to the rotation of the procedure tool. The aforesaid three degrees of freedom can be realized by the transfer of the transfer frame 120 in the front and rear direction, the transfer of the transferring portion 140 in the front and rear direction, and the transfer of the transferring portion 150 in the front and rear direction. The aforesaid two degrees of freedom can be realized by the rotation of the catheter about the rotation axis, and the rotation of the guide wire about the rotation axis. Further, since the transferring portion 130 can be transferred in the front and rear direction with respect to the transfer frame 120, the vascular intervention procedure device 10 according to one embodiment may additionally have one degree of freedom related to the insertion and movement of the procedure tool. Further, since the micro catheter can be rotated by the micro catheter module 400 coupled to the transferring portion 140, the vascular intervention procedure device 10 according to one embodiment may additionally have one degree of freedom related to the rotation of the procedure tool.

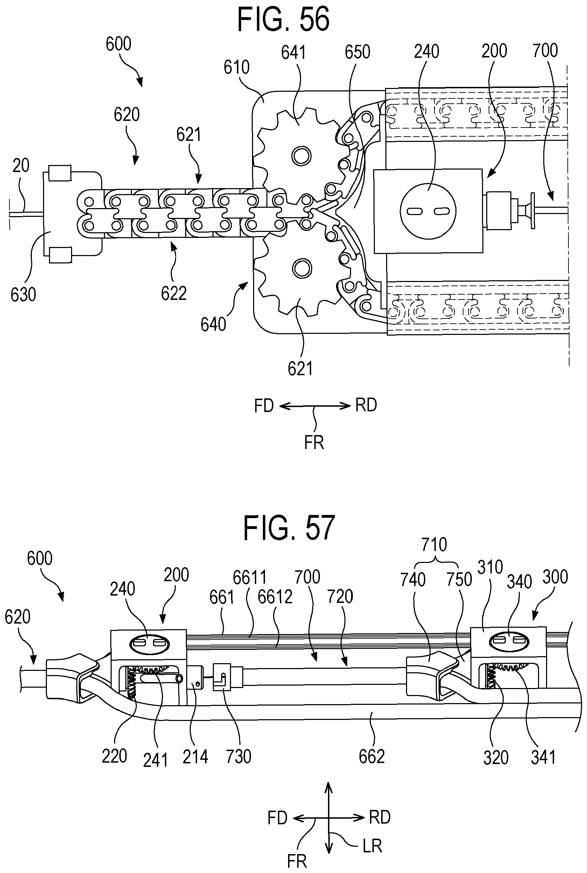

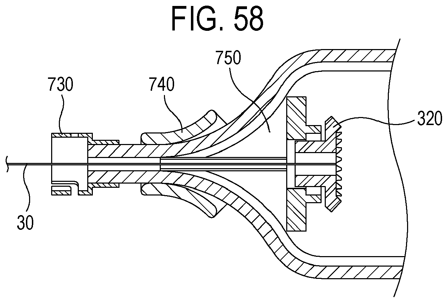

With reference to FIGS. 53 to 59, another embodiment of the vascular intervention procedure device is described. FIG. 53 schematically shows a configuration of a vascular intervention procedure device according another embodiment. FIGS. 54, 55, and 56 show the micro catheter module, the guide wire module, and the first guide module of the vascular intervention procedure device according to another embodiment, respectively. FIG. 57 shows the first guide module and the second guide module of the vascular intervention procedure device according to another embodiment, and FIG. 58 shows a cross-sectional shape of the second guide module shown in FIG. 57.

Referring to FIG. 53, in the vascular intervention procedure device 10 according to another embodiment, the transfer frame 120 of the transfer module 102 is transferred in the front and rear direction FR with respect to the base frame 110 of the platform 100. The frame slider 113 is coupled to the frame transferring lead screw 111 so as to be transferred through screw motion, and the transfer frame 120 is coupled to the frame slider 113.

The catheter module 200 is configured to rotate the catheter 20, and is separably coupled to the transferring portion 130 of the transfer frame 120. The transferring portion 130 is disposed at the front end of the transfer frame 120. The transferring portion 130 is fixed to the transfer frame 120, and is transferred in the front and rear direction by the transfer of the transfer frame 120 in the front and rear direction FR. The transferring portion 130 has the rotation power generating portion 160, and transmits the rotation power rotating the catheter to the catheter module 200 through the power transmitting portion 170. The power transmitting portion 170 is separably coupled to the rotation power generating portion 160. The catheter module 200 includes, in the first module housing 210, the first driven gear 220 coupled to the catheter 20. The catheter module 200 includes a rotation power transmitting portion 240, and the rotation power transmitting portion 240 includes a rotation power transmitting gear 241 transmitting the rotation power to the first driven gear 220. The rotation power transmitting gear 241 receives the rotation power from the power transmitting portion 170 of the transferring portion 130.

The transferring portion 140 is coupled to the module transferring lead screw 123 so as to be transferred through screw motion. The transferring portion 140 is transferred independently of the transferring portion 130. The transferring portion 140 includes the rotation power generating portion 160 in the vicinity of the rear end of the transferring portion 140, and the power transmitting portion 170 separably coupled to the rotation power generating portion 160 and transmitting the rotation power. The guide wire module 300 is configured to rotate the guide wire 30 inserted into the catheter 20, or the micro guide wire 50 inserted into the micro catheter 40. The guide wire module 300 includes, in the second module housing 310, the second driven gear 320 for rotating the guide wire. The guide wire module 300 includes a rotation power transmitting portion 340, and the rotation power transmitting portion 340 includes a rotation power transmitting gear 341 transmitting the rotation power to the second driven gear 320. The rotation power transmitting gear 341 receives the rotation power from the power transmitting portion 170 of the transferring portion 140.

Further, the micro catheter module 400 is separably coupled to the front end of the transferring portion 140. The micro catheter module 400 can be transferred in the front and rear direction independently of the transferring portion 130 and the catheter module 200. The micro catheter module 400 may be separably coupled to a holder device extending from the transferring portion 140. Referring to FIGS. 53 and 54, the micro catheter module 400 includes the third module housing 410, and a guide pipe 430 fitted to the third module housing 410 coaxially with the rotation axis RA. The guide pipe 430 may be configured to fix the micro catheter 40 at its rear end.

The transferring portion 150 may be provided in the transferring portion 140. The transferring portion 140 includes a lead screw 144 which is driven so as to be rotated and is disposed in the front and rear direction FR. The transferring portion 150 may be coupled to the lead screw 144 so as to be transferred through screw motion. The transferring portion 150 may be transferred independently of the transferring portion 130 and the transferring portion 140.

A wire transferring module 900 of the guide wire module 300 is separably coupled to the transferring portion 150. The wire transferring module 900 may be separably coupled to a holder device extending from the transferring portion 150. The wire transferring module 900 is configured to support the guide wire or the micro guide wire. Referring to FIG. 55, the wire transferring module 900 includes a fifth module housing 910, and a guide pipe 940 fitted to the fifth module housing 910 coaxially with the rotation axis RA. The guide pipe 940 may be configured to fix the guide wire or the micro guide wire at its rear end.

In the vascular intervention procedure device of this embodiment, the guide wire 30 and the micro guide wire 50 can be rotated by the guide wire module 300. Referring to FIG. 55, the central portion of the second driven gear 320 is formed with the through hole 321, and a rotating plate 350 is coupled to the second driven gear coaxially. A through hole 351, which corresponds to the through hole of the second driven gear, is formed in the rotating plate 350. The guide pipe 940 of the wire transferring module 900 passes through the through hole 321 and the through hole 351. The guide wire module 300 includes a flexible guide tube 360, both ends of which are coupled to the guide pipe 940 and the rotating plate 350, respectively. One end of the guide tube 360 is coupled to the guide pipe 940, and the opposite end of the guide tube 360 is coupled to the rotating plate 350 eccentrically with the rotation axis RA.

The guide wire 30 or the micro guide wire 50 passes through the guide tube 360 and the guide pipe 940. The guide wire 30 or the micro guide wire 50 may be disposed in the guide pipe 940 coaxially with the rotation axis RA. One end of the guide wire 30 is coupled to the rotating plate 350, and the guide wire is inserted into the catheter. One end of the micro guide wire 50 is coupled to the rotating plate 350, and the micro guide wire is inserted into the micro catheter 40. As the second driven gear 320 is rotated by the rotation power from the transferring portion 140, the rotating plate 350 is rotated together with the second driven gear 320. Since one end of the guide tube 360 is spaced apart from the rotation axis RA, the guide wire 30 or the micro guide wire 50 can be rotated by the rotation of the rotating plate 350.

As shown in FIG. 53, the vascular intervention procedure device 10 may include the first guide module 600, which guides and supports the transfer of the catheter (or the micro catheter inserted into the catheter) in the guide section between the front end of the platform 100 (the front end of the base frame) and the catheter module 200.

Referring to FIG. 56, the first guide housing 610 of the first guide module 600 is coupled to the catheter module 200. The pair of first supporting members 620 may be comprised of the above-described first and second chain assemblies 621 and 622. The pair of first supporting members 620 are drawn into and drawn out from the first guide housing 610. The first engaging portion 640, which is configured to cause the pair of first supporting members 620 to be engaged with each other, is disposed in the first guide housing 610, and the first engaging portion 640 includes the pair of first engaging wheels 641. The first connector 630 is fixed to the end portions of the pair of first supporting members 620 in the drawn-out direction. The first connector 630 may be separably coupled to the front end of the platform (the front end of the base frame).

The first guide module 600 may include the splitting portion 650, which is configured to split the pair of first supporting members 620 when the pair of first supporting members 620 are drawn into the first guide housing 610. The splitting portion 650 is disposed between the pair of first supporting members 620 in the first guide housing 610, and may be configured to allow the catheter 20 to pass through the splitting portion in the front and rear direction FR. As shown in FIG. 56, the splitting portion 650 may be formed as a member having a wedge shape.

As shown in FIG. 53, the vascular intervention procedure device 10 may include the second guide module 700, which guides and supports the micro catheter (or the micro guide wire inserted into the micro catheter) in the guide section between the catheter module 200 and the micro catheter module 400. The second guide module 700 may be comprised of a guide pipe, which is disposed between the catheter module 200 and the micro catheter module 400 and can be inserted into the guide pipe 430 of the micro catheter module 400.

In the vascular intervention procedure device 10, the first guide module and the second guide module may be configured similarly to the first and second bands of the above-described embodiment. In this regard, reference is made to FIGS. 57 and 58.

The first guide module 600 and the second guide module 700 include a pair of supporting members which are drawn into and drawn out from the guide housing according to the length change of the guide section while guiding and supporting the transfer of the procedure tool. The pair of first supporting members 620 of the first guide module 600 and the pair of second supporting members 720 of the second guide module 700 may be configured similarly to the above-described first and second bands. For example, the pair of first supporting members 620 comprise a first band 661 and a second band 662, which are configured to be engaged with each other in the transverse direction LR. The first and second bands 661 and 662 of this embodiment may have a semi-circular cross-sectional shape. The first band 661 has, in its surface facing the second band 662 , an engagement protrusion 6611 and an engagement groove 6612 formed in the front and rear direction FR. The second band 662 has, in its surface facing the first band 661, an engagement groove corresponding to the engagement protrusion 6611 of the first band, and an engagement protrusion corresponding to the engagement groove 6612 of the first band.

The second guide housing 710 of the second guide module is coupled to the second module housing 310 of the guide wire module, and includes the second engaging portion 740 and a splitting portion 750. The second engaging portion 740 and the splitting portion 750 may be formed integrally. As the pair of second supporting members 720 are drawn out from the second guide housing 710, the second engaging portion 740 makes contact with the pair of second supporting members 720 and causes the pair of second supporting members 720 to be engaged with each other. A rear end of the second engaging portion 740 is formed to be broader than a front end thereof, and the splitting portion 750 is located in the rear end of the second engaging portion 740. The splitting portion 750 may have a wedge shape. When the pair of second supporting members 720 are drawn into the second guide housing 710, the splitting portion can split the pair of second supporting members 720. The second guide module includes the second connector 730 which fixes the end portions of the pair of second supporting members 720 in the drawn-out direction. The second connector 730 may be configured similarly to the above-described second connector, and may be separably coupled to the connecting portion 214 formed at the rear end of the first module housing of the catheter module 200. The first guide module 600 may have the same configuration as the configuration of the second guide module 700. In the first guide module 600, the first connector, which fixes the end portions of the pair of first supporting members 620 in the drawn-out direction, may be separably fixed to the front end of the platform (the front end of the base frame).

The technical idea of the present disclosure has been described heretofore with reference to some embodiments and examples shown in the accompanying drawings. However, it is to be understood that various substitutions, modifications, and alterations may be made without departing from the technical idea and scope of the present disclosure that can be understood by those of ordinary skill in the technical field to which the present disclosure pertains. Further, it is to be understood that such substitutions, modifications, and alterations fall within the scope of the appended claims.

## Claims

1. A vascular intervention procedure device, comprising:
a base frame extending in a front and rear direction;
a transfer module including a transfer frame coupled to the base frame so as to be transferred in the front and rear direction along the base frame;
a first procedure tool module coupled to the transfer module so as to be transferred with respect to the base frame in the front and rear direction and configured to rotate a first procedure tool, which is a catheter, about a rotation axis of the front and rear direction; and
a second procedure tool module coupled to the transfer module so as to be transferred with respect to the base frame in the front and rear direction independently of the first procedure tool module and configured to support a second procedure tool which is a guide wire or a micro catheter inserted into the catheter.

2. The vascular intervention procedure device of Claim 1, wherein the transfer module includes:
a first transferring portion disposed in the transfer frame and configured to be coupled to the first procedure tool module and to transmit rotation power for rotating the first procedure tool to the first procedure tool module; and
a second transferring portion disposed in the transfer frame so as to be transferred independently of the first transferring portion in rear of the first transferring portion, and coupled to the second procedure tool module.

3. The vascular intervention procedure device of Claim 2, wherein the transfer module includes a module transferring lead screw disposed along the front and rear direction and coupled to the second transferring portion so as to transfer the second transferring portion through screw motion.

4. The vascular intervention procedure device of Claim 1, wherein the second procedure tool module is a micro catheter module configured to support the micro catheter, and
wherein the vascular intervention procedure device further comprises a third procedure tool module coupled to the transfer module so as to be transferred with respect to the base fame in the front and rear direction and configured to rotate a third procedure tool, which is a micro guide wire inserted into the micro catheter, about the rotation axis.

5. The vascular intervention procedure device of Claim 4, wherein the transfer module further includes:
a first transferring portion disposed in the transfer frame and configured to be coupled to the first procedure tool module and to transmit rotation power for rotating the first procedure tool to the first procedure tool module;
a second transferring portion disposed in the transfer frame so as to be transferred independently of the first transferring portion in rear of the first transferring portion, and coupled to the second procedure tool module; and
a third transferring portion disposed in the transfer frame so as to be transferred in the front and rear direction independently of the first transferring portion and the second transferring portion in rear of the second transferring portion, and configured to be coupled to the third procedure tool module and to transmit rotation power for rotating the third procedure tool to the third procedure tool module.

6. The vascular intervention procedure device of Claim 5, wherein the transfer module includes a module transferring lead screw disposed along the front and rear direction and coupled to the second transferring portion and the third transferring portion so as to transfer the second transferring portion and the third transferring portion through screw motion,
wherein the second transferring portion includes a second transferring nut coupled to the module transferring lead screw so as to be transferred through screw motion, and a second transferring motor configured to rotate the second transferring nut, and
wherein the third transferring portion includes a third transferring nut coupled to the module transferring lead screw so as to be transferred through screw motion, and a third transferring motor configured to rotate the third transferring nut.

7. The vascular intervention procedure device of Claim 6, wherein the first transferring portion includes a transferring nut coupled to the module transferring lead screw so as to be transferred through screw motion.

8. The vascular intervention procedure device of Claim 2, wherein the first transferring portion includes a rotation power generating portion generating rotation power for rotating the first procedure tool, and a power transmitting portion configured to be connected to the rotation power generating portion, to be coupled to the first procedure tool module, and to transmit the rotation power for rotating the first procedure tool.

9. The vascular intervention procedure device of Claim 2, wherein the first transferring portion is disposed in the transfer frame so as to be transferable in the front and rear direction, and
wherein the transfer module is configured to transfer the first transferring portion and the second transferring portion simultaneously or independently.

10. The vascular intervention procedure device of Claim 2, wherein the second procedure tool module is configured to rotate the second procedure tool about the rotation axis, and
wherein the second transferring portion is configured to transmit rotation power for rotating the second procedure tool to the second procedure tool module.

11. The vascular intervention procedure device of Claim 1, further comprising a guide module disposed in a guide section, which is a section between a front end of the base frame and the first procedure tool module or a section between the first procedure tool module and the second procedure tool module, and configured to guide and support transfer of the first procedure tool or the second procedure tool in the front and rear direction,
wherein the guide module includes:
a guide housing disposed at a front end or a rear end of the guide section; and
a pair of supporting members configured to be drawn into or drawn out from the guide housing so as to have a variable length in the guide section according to a length change of the guide section in the front and rear direction, and configured to sandwich the first procedure tool or the second procedure tool by being engaged with each other.

12. The vascular intervention procedure device of Claim 11, wherein the guide module further includes an engaging portion disposed in the guide housing and configured to make contact with each of the pair of supporting members and to cause the pair of supporting members to be engaged with each other when the pair of supporting members are drawn out from the guide housing.

13. The vascular intervention procedure device of Claim 12, wherein the engaging portion includes:
a pair of engaging wheels rotated by making contact with the pair of supporting members, respectively, and causing the pair of supporting members to be engaged with each other; and
a pair of springs pressing the pair of engaging wheels, respectively, in a direction where the pair of supporting members are drawn into the guide housing.

14. The vascular intervention procedure device of Claim 13, wherein the guide housing includes a pair of spring shafts coupled to the pair of springs, respectively, and configured to adjust restoring forces of the pair of springs, respectively.

15. The vascular intervention procedure device of Claim 11, wherein the guide module further includes a splitting portion disposed between the pair of supporting members in the guide housing and configured to split the pair of supporting members when the pair of supporting members are drawn into the guide housing.

16. The vascular intervention procedure device of Claim 11, wherein the pair of supporting members include a first chain assembly having engagement teeth and engagement grooves which are disposed alternately, and a second chain assembly having engagement grooves and engagement teeth which correspond to the engagement teeth and the engagement grooves of the first chain assembly, respectively.

17. The vascular intervention procedure device of Claim 11, wherein the pair of supporting members include a first band having an engagement protrusion formed in the front and rear direction, and a second band having an engagement groove into which the engagement protrusion is fitted.

18. The vascular intervention procedure device of Claim 11, wherein the guide module comprises:
a first guide module disposed in a first guide section between the front end of the base frame and the first procedure tool module and configured to guide and support transfer of the first procedure tool in the front and rear direction; and
a second guide module disposed in a second guide section between the first procedure tool module and the second procedure tool module and configured to guide and support transfer of the second procedure tool in the front and rear direction.

19. The vascular intervention procedure device of Claim 4, further comprising a guide module disposed in a guide section, which is a section between a front end of the base frame and the first procedure tool module, a section between the first procedure tool module and the second procedure tool module, or a section between the second procedure tool module and the third procedure tool module, and configured to guide and support transfer of the first procedure tool, the second procedure tool, or the third procedure tool in the front and rear direction,
wherein the guide module includes:
a guide housing disposed at a front end or a rear end of the guide section; and
a pair of supporting members configured to be drawn into or drawn out from the guide housing so as to have a variable length in the guide section according to a length change of the guide section in the front and rear direction, and configured to sandwich the first procedure tool, the second procedure tool, or the third procedure tool by being engaged with each other.

20. The vascular intervention procedure device of Claim 1, further comprising a transfer module driving portion transferring the transfer module with respect to the base frame in the front and rear direction,
wherein the transfer module is slidably coupled to the base frame by the transfer module driving portion.
